(19) Europäisches Patentamt
European Patent Office
Office européen des brevets

(11) Publication number: **0 284 202 B1**

(12) **EUROPEAN PATENT SPECIFICATION**

(45) Date of publication of patent specification: **10.03.93** (51) Int. Cl.[5]: **C07D 233/80**, A61K 31/415

(21) Application number: **88301474.8**

(22) Date of filing: **22.02.88**

(54) **Substituted hydantoin compounds.**

(30) Priority: **23.02.87 GB 8704158**

(43) Date of publication of application:
**28.09.88 Bulletin 88/39**

(45) Publication of the grant of the patent:
**10.03.93 Bulletin 93/10**

(84) Designated Contracting States:
**AT BE CH DE ES FR GB GR IT LI LU NL SE**

(56) References cited:
**EP-A- 0 126 849**

(73) Proprietor: **THE WELLCOME FOUNDATION LIMITED**
**Unicorn House 160 Euston Road**
**London NW1 2BP(GB)**

(72) Inventor: **Robertson, Alan Duncan**
**THE WELLCOME FOUNDATION LIMITED**
**Langley Court**
**Beckenham Kent(GB)**
Inventor: **Kelly, Michael Gerard**
**THE WELLCOME FOUNDATION LIMITED**
**Langley Court**
**Beckenham Kent(GB)**
Inventor: **Wallace, Paul Neil**
**THE WELLCOME FOUNDATION LIMITED**
**Langley Court**
**Beckenham Kent(GB)**
Inventor: **Giles, Heather**
**THE WELLCOME FOUNDATION LIMITED**
**Langley Court**
**Beckenham Kent(GB)**
Inventor: **Leff, Paul**
**THE WELLCOME FOUNDATION LIMITED**
**Langley Court**
**Beckenham Kent(GB)**
Inventor: **Stepney, Raymond James**
**THE WELLCOME FOUNDATION LIMITED**
**Langley Court**
**Beckenham Kent(GB)**
Inventor: **Singaram, Gonapushnie**
**THE WELLCOME FOUNDATION LIMITED**
**Langley Court**
**Beckenham Kent(GB)**

(74) Representative: **Garrett, Michael et al**
**The Wellcome Research Laboratories Group**
**Patents and Agreements Langley Court**
**Beckenham Kent BR3 3BS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 284 202 B1

## Description

This invention relates to novel substituted hydantoin derivatives suitable for use as therapeutic agents and useful as prostenoid receptor probes. The compounds described herein have chemical and pharmacological properties related to those of natural prostaglandins as demonstrated by their ability to antagonise the physiological effects of prostaglandin $D_2$ ($PGD_2$) in various biological preparations.

In GB-A-1595694, GB-A-1595695, GB-A-1603407 and EP-A-0126849 are described certain hydantoin derivatives which have pharmacological properties related to those of natural prostaglandins. However, none of the hydantoin derivatives described therein have specific selectivity for a particular class of prostacyclin receptors over other prostanoid receptor types.

We have now discovered a novel class of hydantoin derivatives, shown below in formula (I), which are selective antagonists of the $PGD_2$ DP-receptor (nomenclature of I Kennedy et al, Prostaglandins, 24, 667 (1982)) and which have useful medical prophylactic and therapeutic properties for the treatment of disease and disease conditions wherein blockage of the DP-receptor mediated effects of $PGD_2$ is indicated, as well as certain non-medical uses such as providing a prostanoid receptor probe specific for $PGD_2$ receptors which can be used in diagnostic applications.

According to a first aspect of the present invention, therefore, there is provided a compound of formula (I)

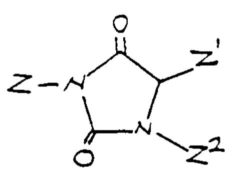

(I)

wherein

Z is $C_{3-12}$ alkenyl or alkynyl, $C_6$ or $C_{10}$ aryl, $C_6$ or $C_{10}$ aryl-$C_{1-12}$ alkyl (wherein in either case the aryl group is optionally substituted by one or more groups independently selected from phenyl, $C_{1-4}$ alkyl, $C_{1-4}$ alkoxy, nitro, and halo) or $C_{4-8}$ cycloalkyl-$C_{1-12}$ alkyl;

$Z^1$ is a group of formula $-CH_2-X-X^1-X^2$, wherein X is selected from $-(CH_2)_2-$and cis and trans $-CH=CH-$, $X^1$ is a covalent bond or a straight or branched alkylene chain having from 1 to 6 carbon atoms optionally having one of its methylene groups replaced by oxa (-O-) or thia (-S-) provided that at least one carbon atom separates the oxa or thia group from a carboxyl or -CH=CH-group, and $X^2$ is a carboxyl group, a $C_{1-4}$ alkyl ester, or an amide group;

$Z^2$ is a group selected from $-NH-CH_2-R$ and $-N=CH-R$, wherein R is a group selected from -CO-Y and $-CH(Y^1)-Y$, Y being a group selected from $C_{3-8}$ alkyl, $C_{3-8}$ alkenyl, phenyl-$C_{1-4}$ alkyl and phenyl (wherein the phenyl group in both cases is optionally substituted by one or more groups independently selected from $C_{1-4}$ alkyl, $C_{1-4}$ alkoxy, nitro, halo and trihalomethyl), $C_{4-8}$ cycloalkyl, pyridyl, thienyl, tetrahydropyranyl and tetrahydrofuryl; and $Y^1$ being a group selected from hydrogen, hydroxy, $C_{1-4}$ alkoxy and $C_{2-5}$ acyloxy; and salts and solvates thereof.

Unless the context indicates to the contrary, any reference hereinafter to a compound of formula (I) is a reference to the compound of formula (I) per se and to the salts and solvates thereof. When intended for administration to the human or animal body (as described further hereinbelow) such salts and solvates are those which are pharmaceutically acceptable. Furthermore, in formula (I) and throughout this specification, unless specifically stated to the contrary, alkyl groups or the alkyl part of alkyl-containing moieties such as acyl, are preferably straight or branched and contain from one to four carbon atoms. "Halo" means, in particular, chloro, bromo or iodo. When Y is an aralkyl group, this may be a phenalkyl group, for example, benzyl. The above-mentioned salts may be formed from those compounds of formula (I) wherein $X^2$ is a carboxyl group. Particularly valuable salts for medical purposes are those having a physiologically acceptable cation, such as ammonium or that of an alkali metal, for example, sodium or potassium, an alkaline earth metal, for example, calcium or magnesium, or an organic base, particularly an amine such as tri(hydroxymethyl)aminomethane or ethanolamine. Salts having non-physiologically acceptable cations are included within the ambit of this invention as useful intermediates for the isolation or purification of the corresponding compounds of formula (I) or their physiologically acceptable salts or solvates. Except when there is clear indication to the contrary, formula (I) and other formulae in the specification embrace all stereoisomers represented therein. In particular, such formulae include geometrical isomers having the syn

and anti configurations at the -N=CH- group, as well as diastereoisomers and individual enantiomers and racemic mixtures of these forms.

Preferred compounds of formula (I), by virtue of their advantageous pharmacological and other properties, include those wherein:

Z is phenyl or phenyl-$C_{1-3}$ alkyl wherein the phenyl group is optionally substituted by one or more groups independently selected from phenyl, $C_{1-4}$ alkyl and $C_{1-4}$ alkoxy;

$Z^1$ is $-CH_2$-X-$X^1$-$X^2$ wherein X is $-(CH_2)_2-$, $X^1$ is an alkylene chain of from 2 to 4 carbon atoms and $X^2$ a is carboxyl group or a $C_{1-4}$ alkyl ester; and

$Z^2$ is a group selected from $-NH-CH_2-R$ and -N=CH-R wherein R is a group of formula $-CH(Y^1)-Y$ wherein $Y^1$ is hydroxy and Y is phenyl, $C_{3-8}$ alkyl or $C_{4-8}$ cycloalkyl, particularly cyclohexyl;

and salts and solvates thereof.

Particularly preferred compounds, including salts and solvates thereof, are 3-benzyl-5-(6-carboxyhexyl)-1-(2-cyclohexyl-2-hydroxyethylamino)hydantoin and its ethylideneamino analogue 3-benzyl-5-(6-carboxyhexyl)-1-(2-cyclohexyl-2-hydroxyethylideneamino)hydantoin. Of these, 3-benzyl-5-(6-carboxyhexyl)-1-(2-cyclohexyl-2-hydroxyethylamino)hydantoin is especially preferred by virtue of its exceptional pharmacological properties.

As indicated, the compounds of formula (I) have chemical and pharmacological properties related to those of natural prostaglandins. Specifically, the compounds of the invention antagonise some of the physiological effects of $PGD_2$ and are therefore particularly useful in the treatment or prophylaxis of diseases or disease conditions wherein $PGD_2$ is overproduced. For example, some of the symptoms exhibited by individuals having diseases or disease conditions involving mast cell disfunction are consistent with the overproduction of $PGD_2$, a compound which is known to be released by the degranulation of mast cells. Diseases or disease conditions for which the present compounds will be particularly useful therefore include those involving mast cell disfunction, such as systemic mastocytosis and systemic mast cell activation disorders, urticaria and allergic rhinitis.

Systemic mastocytosis is a disease in which there is abnormal proliferation of tissue mast cells and extremely high plasma and urinary levels of $PGD_2$ and its metabolites. Patients experience episodic attacks of facial flushing, severe headache, syncope, tachycardia and, sometimes, life-threatening hypotension symptoms which are reminiscent of the effects of $PGD_2$ infusion in normal men.

Heat and cold induced urticaria are associated with dermal mast cell degranulation and are accompanied by itching, erythema and oedema. $PGD_2$may be involved in the skin reactions mediated by mast cell degranulation either by direct action or by potentiating the effects of other mediators. Patients with allergic rhinitis also release histamine and $PGD_2$ into nasal secretions following challenge with antigen. The nasal stuffiness associated with this condition is the result of $PGD_2$-mediated vasorelaxation.

According to a second aspect of the present invention, therefore, there is provided a compound of formula (I) for use in a method of treatment or prophylaxis of the human or animal body by therapy, particularly the treatment or prophylaxis of diseases or disease conditions wherein blockade of the DP-receptor medicated effects of $PGD_2$ is indicated, for example, conditions in which overproduction of $PGD_2$ is implicated, such as systemic mastocytosis and systemic mast cell activation disorders, urticaria and allergic rhinitis.

As also indicated, the compounds of formula (I) provide a prostanoid receptor probe which is specific for $PGD_2$ receptors over other prostanoid receptor types. This ability of the compounds of the invention to selectively block $PGD_2$ receptors finds particular utility in pharmacological assays such as those used for diagnostic purposes. For example, the present compounds may be used with advantage in research on prostaglandin mimetics other than $PGD_2$ where specific blockade of $PGD_2$ receptors is required.

According to a third aspect of the present invention, therefore, there is provided a compound of formula (I) for use as a prostanoid receptor probe specific for $PGD_2$ receptors.

The amount of a compound of formula (I) required to achieve the desired therapeutic effect will, of course, depend on a number of factors, for example, the specific compound chosen, the disease of disease condition for which it is intended, the mode of administration, and the recipient. In general, a daily dose may be expected to lie in the range 0.01mg to 500mg per kilogram bodyweight. For example, an intravenous dose may lie in the range 0.01mg to 100mg/kg which may conveniently be administered as an infusion of from 1.0$\mu$g to 100$\mu$g/kg/minute. Infusion fluids suitable for this purpose may contain, for example, from 1.0$\mu$g to 1.0mg per millilitre.

Unit dose formulations may contain from 10$\mu$g to 500mg of active compound according to the mode of administration and may be administered one or more times per day separately or in multiples. Thus ampoules for injection may contain, for example, from 0.1mg to 10mg of active compound, orally administerable formulations, such as tablets or cachets, may contain, for example, from 1mg to 500mg,

typically from 10mg to 100mg, and inhalational doses may contain, for example, from 10µg to 500µg per inhalation.

The amount of a compound of formula (I) required in a diagnostic application to specifically blockade $PGD_2$ receptors will, of course, depend on the particular application and on the nature of the specific compound used. Typically an aqueous solution of the compound having a concentration of from 1nM to 1µM is employed.

The above mentioned doses refer to the compounds of formula (I) per se; where a salt or solvate is used, the dose should be taken as referring to the corresponding anion or unsolvated compound.

According to a fourth aspect of the present invention, there is provided a pharmaceutical formulation comprising, as active ingredient, at least one compound of formula (I) and/or a pharmacologically acceptable salt or solvate thereof, together with at least one pharmaceutical carrier or excipient. These pharmaceutical formulations may be used in the treatment or prophylaxis of any of the diseases or disease conditions referred to above wherein blockade of the DP-receptor mediated effects of $PGD_2$ is indicated. The carrier must of course be 'acceptable' in the sense of being compatible with the other ingredients of the formulation and not be deleterious to the recipient. The carrier maybe a solid or a liquid, and is preferably formulated with a compound of formula (I) as a unit dose formulation, for example, a tablet which may contain from 0.05% to 95% by weight of the active ingredient. Other pharmacologically active substances may also be present in the formulations of the present invention, for example, a histamine antagonist such as acrivastine or triprolidine. The compound of formula (I) may be incorporated in the formulations of the invention as the compound per se or as a pharmaceutically acceptable salt or solvate thereof. The formulations may be prepared by any of the well-known techniques of pharmacy consisting essentially of admixture of components of the formulation.

Formulations of the invention include those suitable for administration by oral, buccal (eg sub-lingual), parenteral (eg subcutaneous, intramuscular, or intravenous), or rectal routes, by topical application, or by nasal/buccal inhalation. In any given case, the most suitable mode of administration will depend on the nature and severity of the condition being treated and on the nature of the active compound used.

Formulations suitable for oral administration may be presented as discrete units, such as capsules, cachets, lozenges, or tablets, each containing a predetermined amount of the active compound; as powders or granules; as solutions or suspensions in aqueous non-aqueous liquids; as oil-in-water emulsions; or as water-in-oil emulsions. Such formulations may be prepared by any of the methods of pharmacy, but all methods include the step of bringing into association the active ingredient with a carrier which comprises one or more appropriate ingredients. In general, the formulation may be prepared by uniformly and intimately admixing the active ingredient with a liquid or finely divided solid carrier, or both, and then, if necessary, shaping the product into the desired presentation. For example, a tablet may be prepared by compressing or moulding a powder or granules of the active ingredient, optionally with one or more accessory ingredients. Compressed tablets may be prepared by compressing, in a suitable machine, the active ingredient in a free-flowing form, such as a powder or granules, optionally mixed with a binder, lubricant, inert diluent, or surface active/dispersing agent(s). Moulded tablets may be made by moulding in a suitable machine the powdered active ingredient moistened with an inert liquid diluent.

Formulations suitable for buccal (eg sub-lingual) administration include lozenges comprising the active ingredient in a flavoured base, eg sucrose and acacia or tragacanth; and pastilles comprising the active ingredient in an inert base such as gelatin and glycerin or sucrose and acacia.

Formulations suitable for parenteral administration conveniently comprise sterile aqueous preparations of the active ingredient, which preparations are preferably isotonic with the blood of the intended recipient. These preparations are preferably administered intravenously, although administration may also be effected by means of subcutaneous or intramuscular injection. Such preparations may conveniently be prepared by admixing the active ingredient with water and rendering the product sterile and isotonic with the blood.

Formulations suitable for rectal administration are preferably presented as unit-dose suppositories. These may be prepared by admixing the active ingredient with one or more conventional solid carriers forming the suppository base, for example, cocoa butter, and shaping the resulting mixture.

Formulations suitable for topical application to the skin preferably take the form of an ointment, cream, lotion, paste, gel, spray, aerosol, or oil. Carriers which may be used in such formulations include petroleum jelly, lanolin, polyethylene glycols, alcohols and combinations thereof. The active ingredient is generally present in a concentration of from 0.1% to 15% w/w of the composition, for example, from 0.5% to 2%.

Formulations suitable for nasal/buccal inhalation include compositions consisting of particles containing active ingredient which can be delivered into the lungs of the patient. Such compositions are conveniently in the form of a dry powder for administration from a powder inhalation device or self-propelling powder-dispensing container, for example, a self-propelling aerosol composition in a sealed container. Preferably,

the powder comprises particles of which at least 98% by weight have a diameter of greater than 0.5μm and at least 95% by number have a diameter of less than 7μm. Most desirably, at least 95% by weight of the particles have a diameter of greater than 1μm and at least 90% by number have a diameter less than 6μm. Compositions in the form of dry powders preferably include a solid fine powder diluent, such as sugar, and are conveniently presented in a pierceable capsule, for example, of gelatin.

Self-propelling compositions in accordance with the invention may be either powder-dispensing compositions or compositions dispensing the active ingredient in the form of droplets of a suspension. Self-propelling powder-dispensing compositions typically includes a liquid propellant having a boiling point of below 18°C at atmospheric pressure. Generally the propellant may constitute 50% to 99.9% w/w of the composition whilst the active ingredient may constitute from 0.1% to 20% w/w, of the composition, for example, about 2% w/w. The carrier in such compositions may include other constituents, in particular a liquid non-ionic or solid anionic surfactant, or a solid diluent (preferably having a particle size of the same order as the particles containing the active ingredient) or both. The surfactant may constitute from 0.01% up to 20% w/w of the composition, though preferably below 1% w/w.

Self-propelling compositions wherein the active ingredient in present in solution comprise an active ingredient, propellant and co-solvent, and advantageously an antioxidant stabiliser. The co-solvents may constitute 5% to 40% w/w of the composition, though preferably less than 20% w/w.

Compositions of the present invention may also be in the form of an optionally sterile aqueous or dilute alcoholic solution of the active ingredient for use in a nebuliser or atomiser, wherein an accelerated air stream is used to produce a fine mist consisting of small droplets of the solution. Such formulations usually contain a flavouring agent, such as saccharin sodium, and a volatile oil. A buffering agent and a surface active agent may also be included in such a formulation, together with a preservative such as methylhydroxybenzoate.

Other formulations suitable for nasal administration include a coarse powder having a particle size of from 20μm to 500μm which is administered in the manner in which snuff is taken, ie by rapid inhalation through the nasal passage from a container of the powder held close to the nose.

In addition to the aforementioned ingredients, the formulations of this invention may include one or more additional ingredients such as diluents, buffers, flavouring agents, binders, surface active agents, thickeners, lubricants, preservatives (including anti-oxidants) and emulsifying agents. Any other therapeutic ingredient may comprise one or more of the following: antibiotic (eg anti-bacterial), anti-fungal and anti-viral agents, and anti-histamines (particularly peripherally acting anti-histamines). However, when such other agent(s) are also present, the compound of formula (I) or physiologically acceptable salt or solvate thereof and the other agent(s), need not necessarily be present as a pharmaceutical formulation as hereinbefore defined, but merely in combination or intimate admixture, ie a pharmaceutically acceptable carrier need not be present.

According to a fifth aspect of the present invention, therefore, there is provided the use of a compound of formula (I) in the preparation of an agent for use in the treatment or prophylaxis of diseases or disease conditions wherein blockade of the DP-reactor mediated effects of $PGD_2$ is indicated.

The compounds of formula (I) may be prepared by any conventional manner, and, in accordance with the present invention, may, for example, be prepared by any method hereinafter described.

Thus according to a sixth aspect of the present invention, we provide a process for the preparation of compounds of formula (I) which comprises

(a) for the preparation of hydantoins wherein $Z^2$ is -N=CH-R, wherein R is as defined above, reacting a compound of formula (II) with a compound of formula (III)

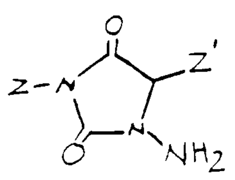

R-HCO (III)

(II)

wherein Z, $Z^1$ and R are as defined above, in, for example, the presence of base and a suitable solvent, such as methanol;

(b) for the preparation of hydantoins wherein $Z^2$ is -N=CH-R, wherein R is as defined above, reacting a compound of formula (IV)

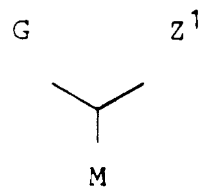

(IV)

wherein $Z^1$ is as defined above, G is carboxy or a derivative thereof, such as an amide derivative, for example, carbamoyl, or an ester thereof, particularly a $C_{1-4}$ alkyl ester thereof, or G is cyano, and M is a leaving group such as halo, preferably bromo, with a compound of formula (V)

ZHNCONHN=CHR (V)

wherein R and Z are as defined above, in the presence of base in a polar solvent, such as ethanol;

(c) for the preparation of hydantoins wherein $Z^2$ is -N=CH-R, wherein R is as defined above, cyclising a compound of formula (VI)

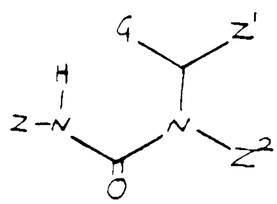

(VI)

wherein G, Z, $Z^1$ and $Z^2$ are as defined above, in, for example, the presence of acid or base or by heating in an inert solvent;

(d) for the preparation of hydantoins wherein $Z^2$ is -N-CH=R, wherein R is as defined above, treating a compound of formula (VII)

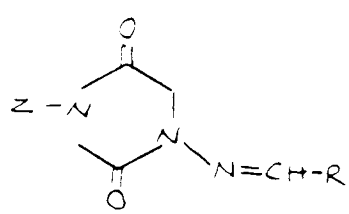

(VII)

wherein R is as defined above, with methyl magnesium carbonate followed by reaction in the presence of base with a compound of formula (VIII)

M-$Z^1$ (VIII)

wherein M and $Z^1$ are as defined above, in a non-protic solvent, such as THF or ether;

(e) reacting a compound of formula (IX) with a compound of formula (X)

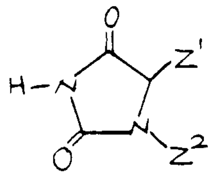

$Z^3$-L (X)

(IX)

wherein $Z^1$ and $Z^2$ are as defined above, L is a suitable leaving group, such as halo (eg bromine) or hydroxy, and $Z^3$ is defined as for Z above (other than $C_6$ or $C_{10}$ aryl) or an alternative imino protecting group, for example, benzylidineamino (-N = CH-Ph), in the presence of base and, when L is hydroxy, triphenylphosphase and diethylazodicarboxylate (Mitsunobu, Tetrahedron Letters (1972) 1279);
and subsequently, if desired, effecting one or more of the following optional conversions in any desired order:
i) wherein a hydantoin of formula (I) is formed and an ester, converting said ester into the corresponding acid or a salt or solvate amide thereof;
ii) where a hydantoin of formula (I) is formed and is an acid, converting said acid into the corresponding ester or a salt or solvate thereof;
iii) where a hydantoin of formula (I) is formed wherein Z is hydrogen, converting said hydantoin into a corresponding hydantoin wherein Z is alkyl;
iv) where a hydantoin of formula (I) is formed wherein R is (-CH($Y^1$)Y, wherein $Y^1$ is an acycloxy group and Y is as defined above, converting said hydantoin into a corresponding hydantoin of formula (I) wherein $Y^1$ is a hydroxy group;
v) where a hydantoin of formula (I) is formed wherein R is -CO-Y, converting said hydantoin into a corresponding hydantoin wherein R is -CH(OH)-Y; or
vi) where a hydantoin of formula (I) is formed wherein $Z^2$ is -N = CH-R, converting said hydantoin into a corresponding hydantoin wherein $Z^2$ is $-NH-CH_2-R$, wherein R is as defined above.

The reaction of process (a) may conveniently be performed in a suitable solvent, for example, methanol.

The reaction of process (b) may conveniently be carried out by heating in the presence of a suitable base, such as an alkali metal alkoxide, for example, sodium ethoxide, and optionally in the presence of a suitable solvent, such as ethanol;

In process (c), the carboxyl derivative may, for example, be an amide or ester, in particular, an alkyl ester. The cyclisation may be performed under acidic conditions or by heating alone. The reaction may be effected in the absence of a solvent or an inert solvent may be used, for example, a hydrocarbon solvent, such as petrol. Alternatively, where G is alkoxycarbonyl, cyclisation may be effected in the presence of a suitable base, for example, an alkoxide, such as sodium ethoxide.

In process (d), the compound of formula (VI) may be in a suitable solvent, such as dimethylformamide.

In process (e), where L is selected form halo, then the compound of formula (IX) may preferably be metalated, for example, by reaction of a compound of formula (IX) with sodium hydride in a suitable solvent, for example, dimethylsulphoxide. Where L is a hydroxy group, so that the compound of formula (IX) is an alcohol, process (e) may employ known conditions for such substitution, for example, the presence of other reagents, such as triphenylphosphine and diethylazodicarboxylate.

If, in the above general processes, it is desired separately isolate one or both diastereoisomers of a compound of formula (I) in the case where an appropriate compound exists in mixed isomeric form, separation of the isomers may conveniently be performed in conventional manner, for example, by h.p.l.c. or by preferential crystallisation.

Compounds of formula (II) may be prepared by hydrogenation of a compound of formula (XI)

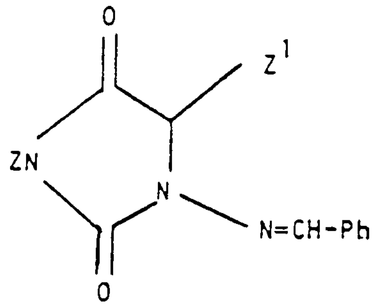

(XI)

wherein Z and $Z^1$ are as defined above and Ph represents phenyl, under suitable conditions. Conveniently, such hydrogenation may be performed at elevated pressure in the presence of a palladium/charcoal catalyst. If desired, the reaction may be carried out with heating.

Compounds of formula (II) may also be prepared by reaction of a compound of formula (XII)

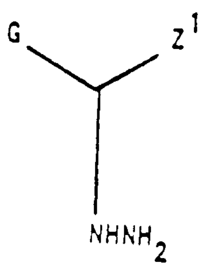

(XII)

wherein G is as defined above, with an isocyanate compound Z-NCO, wherein Z is as defined above. The reaction may proceed in the absence of a solvent, but desirably an inert solvent is used which is preferably polar, such as water or a mixture of water with acetone, dimethylformamide, dimethylsulphoxide, or a lower alkanol, such as ethanol, or a hydrocarbon solvent, an ether, or a halogenated hydrocarbon solvent, such as chloroform. When desired, for example, if no solvent is used, the reaction may be promoted by heating the reactants.

Instead of using isocyante, a compound of formula (X) may be reacted with a suitable urea derivative. A solvent is not essential, but, if desired, an inert solvent, such as one of those mentioned above, may be used; the reaction is preferably effected at an elevated temperature, for example, from 100° to 125°C, but temperatures of up to 150°C may be used.

Compounds of formula (XI) may be prepared by reaction of a compound of formula (IV), as previously defined, with a compound of formula (XIII)

ZHNCONHN=CHPh (XIII)

wherein Z is as defined above and Ph represents phenyl.

Compounds of formulae (V) and (XIII) may be prepared, for example, by reaction of the appropriate aldehyde with semicarbazide, if necessary under mildly acidic conditions.

Compounds of formula (IV) may conveniently be prepared in a manner analogous to that described by Schwank and Papa in J.Amer.Chem.Soc. (1948) 70, 3626.

Compounds of formula (XII) may be prepared by the reaction of a compound of formula (IV), as previously defined, with an excess of hydrazine hydrate under appropriate conditions, in a suitable solvent such as ethanol.

Compounds of formula (III) may be prepared in a manner analogous to that described, for example, by Tiffany et al in J.Amer.Chem.Soc. (1957) 79, 1682, or by Royals and Robinson in J.Amer.Chem.Soc. (1956) 78, 4161.

Compounds of formula (VI) may be prepared by reaction of a compound of formula (XIV)

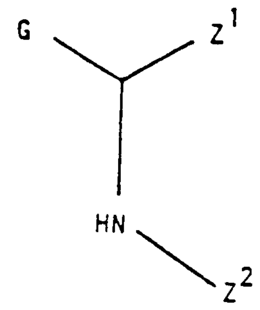

(XIV)

wherein G, $Z^1$ and $Z^2$ are as defined above, with an isocyanate compound Z-NCO, wherein Z is as defined above. The reaction may be performed in a manner, and under conditions, analogous to those previously described for the conversion of a compound of formula (XII) to a compound of formula (II). In the synthesis, the compound of formula (VI) need not be isolated from the reaction mixture and may be converted directly to a compound of formula (I) under the described reaction conditions.

Compounds of formula (XIV) may conveniently be prepared by the reaction of a compound of formula (XII), as previously defined, with a compound of formula (III), as previously defined, under reaction conditions analogous to those described for process (a).

Compounds of formula (VII) may be prepared by the reaction of a compound of formula (XV)

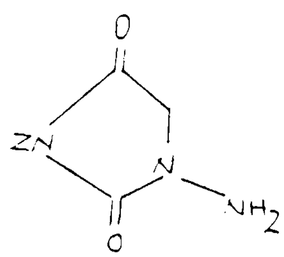

(XV)

with a compound of formula (III), as previously defined, by conventional means.

Compounds of formula (XV) may be prepared according to the method described by D Jack in J.Pharm.Pharmacol. (1959) 108T and, where appropriate, alkylating the ring at the 3-position in a conventional manner.

Compounds of formula (VIII) or (X) may, for example, be prepared in a manner analogous to that described by D E Ames et al in J. Chem.Soc. (1950) p.174. Compounds of formula (IX) may be prepared by the method described in EP-A-0126849.

In optional conversion (i) above, the reaction is advantageously effected by hydrolysis, for example, under basic conditions, such as in the presence of sodium hydroxide. A similar hydrolysis may be employed in optional conversion (iii) where a $Y^1$ acycloxy group is converted into a $Y^1$ hydroxy group.

In optional conversion (ii), the reaction is advantageously effected by treatment with the appropriate alcohol, for example ethanol, in the presence of a suitable acid, such as sulphuric acid.

After either of conversions (i) or (ii), the appropriate carboxylate salt may be formed by treatment of the acid with an appropriate inorganic or organic base in an appropriate solvent, such as water, followed by isolation, for example, by methanol precipitation, evaporation, or freeze-drying.

In conversion (iii), the reaction is advantageously effected by treatment with a suitable alkylating agent, such as an alkyl halide, for example, the iodide, in the presence of a suitable base, such as sodium hydroxide.

In conversion (iv), deacylation may be performed by conventional means, for example, by treatment with sodium hydroxide.

Conversion (v) may be effected by conventional reduction using an agent such as sodium borohydride under basic conditions.

In optional conversion (vi), the reaction may be effected using any appropriate reducing agent, such as the borohydride or cyanoborohydride of an alkali metal, for example, sodium or lithium. The reaction

proceeds under suitable conditions which may, in the case of cyanoborohydrides, require the presence of an acid, for example, acetic acid.

In general, the reactions and conversions specified above may be effected by conventional means using techniques known for the preparation of analogous compounds.

Intermediate compounds of formulae (II), (V), (VI), (VII), (X), (XI), (XIII) and (XV) represent further aspects of the invention.

In all of the foregoing chemical procedures, it is, of course, evident that the choice of reactants will be dictated in part by the functional groups present in the substrates, and, where necessary, reactants having an appropriate selectivity of action must be used.

The following Examples illustrate the invention. In the Examples, reference will be made to various compounds whose structures and exemplary preparative interrelationship are shown in Chart I.

Chart I

$EtO_2CCH(Br)(CH_2)_6CO_2Et$ (IX)

$H_2NCONHN{=}CHPh$ (V/XIII)

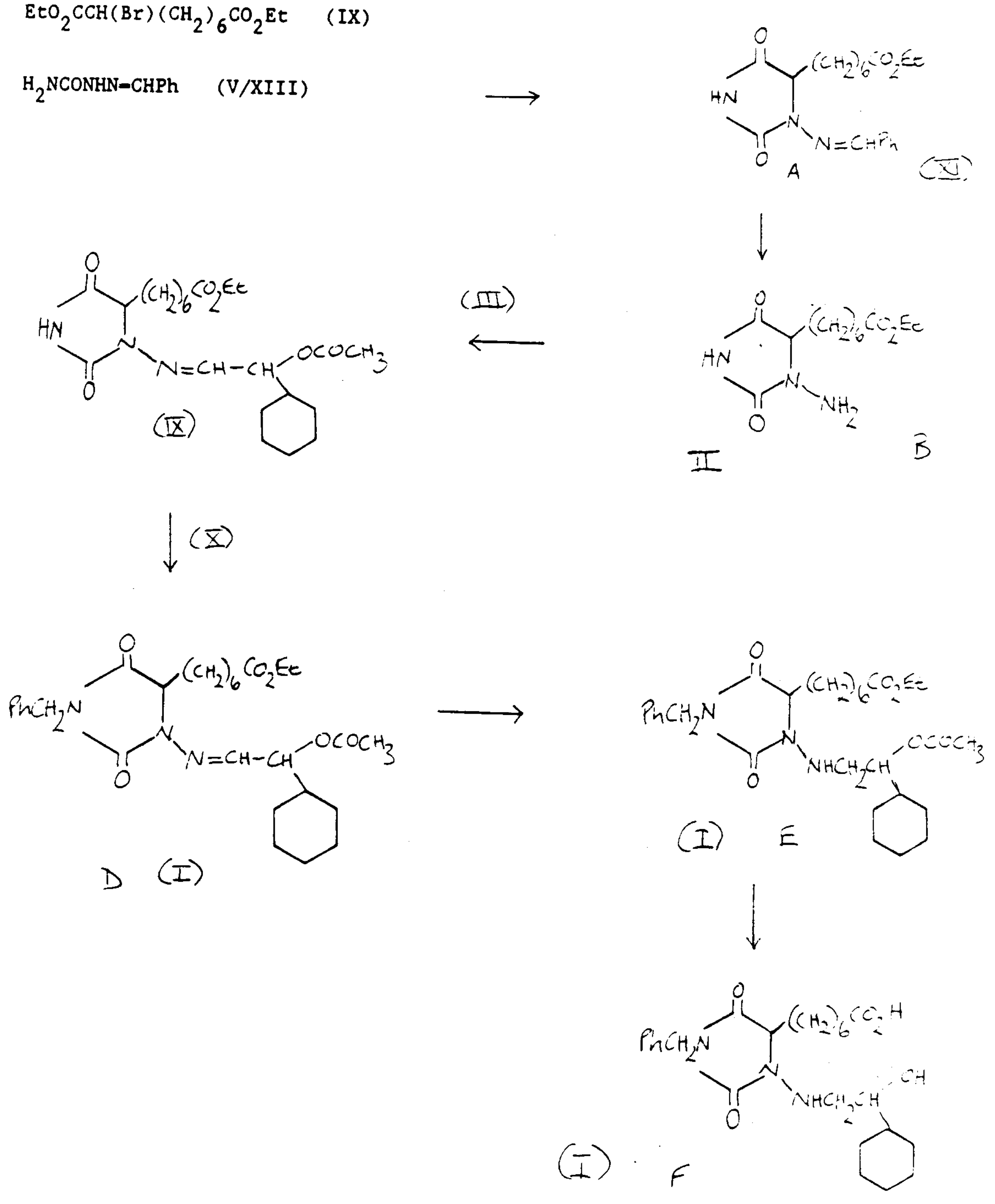

Example 1

3-Benzyl-5-(6-ethoxycarbonylhexyl)-1-(2-acetoxy-2-cyclohexylethylideneamino)hydantoin (Compound D)

(a) 1-Benzylideneamino-5-(6-ethoxycarbonylhexyl)hydantoin (Compound A)

A solution of sodium ethoxide was prepared by dissolving sodium (4.6g) in ethanol (100ml). A suspension of benzaldehyde semicarbazone (16.3g) in the sodium ethoxide solution (50ml) and ethanol (20ml) was refluxed for 15 minutes. Diethyl 2-bromononanedioate (16g) was added and the mixture was refluxed for 30 minutes. Sodium ethoxide solution (25ml) was added and the mixture was refluxed for 5 minutes before the additon of the bromodiester (8g), followed by 30 minutes' refluxing. The remaining sodium ethoxide solution (25ml) and bromodiester (8g) were then added and the mixture was refluxed for 1 hour. Most of the ethanol was evaporated in vacuo and the residue was shaken with dilute hydrochloric acid and ether. Unchanged benzaldehyde semicarbazone (5.4g) was filtered off and the ether solution was washed with water, dried ($MgSO_4$) and evaporated. The semi-solid residue was treated with a small volume of ether to give 1-benzylideneamino-5-(6-ethoxycarbonylhexyl)hydantoin (13.0g) which crystallised from cyclohexane in colourless needles, m.p. 92-94 °C.

(b) 1-Amino-5-(6-ethoxycarbonylhexyl)hydantoin (Compound B)

The above benzylideneamino compound (5.8g) in solution in ethanol (100ml) was hydrogenated at 10 atmospheres' pressure and 50 °C in the presence of 10% palladium/charcoal catalyst for 1 hour. The catalyst was filtered off and the solvent was evaporated to leave the amino compound (4.3g) which crystallised form ethyl acetate/light petroleum (b.p. 60-80 °C) as colourless needles, m.p. 80-82 °C.

(c) 1-(2-Acetoxy-2-cyclohexylethylideneamino)-5-(6-ethoxycarbonyl hexyl)hydantoin (Compound C)

A solution of 1-amino-5-(6-ethoxycarbonylhexyl)hydantoin (3.0g) and 2-acetoxy-2-cyclohexylacetaldehyde (Ross et al, J.Med.Chem. (1979) 22, 412) (2.24g) in methanol (20ml) was refluxed for 1 hour. The methanol was evaporated, the residue was dissolved in ether and the ether solution was washed with 1N hydrochloric acid, then water, and dried. The oil remaining after evaporation of the ether was purified by chromatography on a column of silica using a mixture of chloroform and methanol (40:1) as eluant to give a colourless oil (4.9g) showing two spots, Rf 0.46 and Rf 0.50 on t.l.c. (silica: chloroform-methanol-acetic acid (95:4:1)). Separation of a portion (1.5g) by h.p.l.c. (silica; dichloromethane-methanol-acetic acid (98.5:1.0:0.5)) gave the individual diastereoisomers of 1-(2-acetoxy-2-cyclohexyl-ethylideneamino)-5-(6-ethoxycarbonylhexyl) hydantoin as colourless viscous oils, Rf 0.50 (625mg) and Rf 0.46 (850mg).

(d) 3-Benzyl-5-(6-ethoxycarbonylhexyl)-1-(2-acetoxy-2-cyclohexyl ethylideneamino)hydantoin (Compound D)

A solution of 5-(6-ethoxycarbonylhexyl)-1-(2-acetoxy-2-cyclohexylethylidene amino)hydantoin (868mg), benzyl alcohol (160mg) and triphenylphosphine (530mg) in tetrahydrofuran was treated with diethylazodicarboxylate (0.34 ml) at 15 °C under an atmosphere of nitrogen. After stirring for 20 hours, the solvent was removed in vacuo and the mixture purified by column chromatography (silica gel). Eluting with chloroform gave 3-benzyl-5-(6-ethoxycarbonylhexyl)-1-(2-acetoxy-2-cyclohexylethylideneamino)hydantoin (800mg). $^1$H nmr ($CDCl_3$/delta): 3-benzyl-5-(6-ethoxycarbonylhexyl)-1-(2-acetoxy-2-cyclohexylethylideneamino)-hydantoin 8.2 (1H, N-C<u>H</u>); 7.3 (5H, Ph); 5.1 (1H, C<u>H</u> (OAc)); 4.7 (2H, C<u>H</u>$_2$Ph); 2.05 (3H, COC<u>H</u>$_3$).

Example 2

3-Benzyl-5-(6-ethoxycarbonylhexyl)-1-(2-acetoxy-2-cycohexylethylamino) hydantoin (Compound E)

A solution of 3-benzyl-5-(6-ethoxycarbonylhexyl)-1-(2-acetoxy-2-cyclohexyl ethylideneamino)hydantoin (490mg) in ethanol (5 ml) containing acetic acid (1ml) was treated at 15 °C with sodium cyanoborohydride (72mg). After 20 hours, the solvent was removed in vacuo, water (20 ml) was added and the product extracted into chloroform (20 ml). The extract was dried over magnesium sulphate, filtered and concentrated.

Column chromatography (silica gel: diethyl ether/light petroleum, 1:1) gave 3-benzyl-5-(6-ethoxycarbonylhexyl)-1-(2-acetoxy-3-cyclohexylethylamino) hydantoin (440mg). $^1$H nmr ($CDCl_3$/delta): 7.3 (5H, Ph); 4.8 (1H, C<u>H</u> (OAc)); 4.6 (2H, C<u>H</u>$_2$Ph); 4.3 (1H, N<u>H</u>); 4.1 (2H, $CO_2$Et); 3.4-2.9 (2H, N-$CH_2$).

Example 3

3-Benzyl-5-(6-carboxylhexyl)-1-(2-hydroxy-2-cyclohexylethylamino) hydantoin (Compound F)

A solution of 3-benzyl-5-(6-ethoxycarbonylhexyl)-1-(2-acetoxy-2-cyclohexyl ethylamino)hydantoin (440mg) in ethanol (20 ml) was treated by the dropwise addition of sodium hydroxide (0.2N, 5 ml). After stirring at 10°C for 20 hours, the solution was concentrated *in vacuo*, water (20 ml) was added and the mixture acidified with hydrochloric acid (0.2N). The product was extracted into diethyl ether (20 ml). After drying over magnesium sulphate, filtration and concentration *in vacuo* gave 3-benzyl-5-(6-carboxylhexyl)-1-(2-hydroxy-2-cyclohexylethylamino)hydantoin (450mg).
$^1$H nmr ($CDCl_3$/delta): 7.3 (5H, Ph); 4.6 (2H, C<u>H</u>$_2$Ph); 4.0 (1H, C<u>H</u>N); 3.4 (1H, C<u>H</u>(OH)); 3.0 and 2.8 (1H each, NH-C<u>H</u>$_2$).

Example 4

3-(4-Phenylbenzyl)-5-(6-carboxyhexyl)-1-(2-hydroxy-2-cyclohexylethylamino) hydantoin

By analogy with the combined procedures of Examples 1, 2 and 3, the above compound was prepared.
$^1$H nmr ($CDCl_3$/delta): 7.5 (9H, m, Ph-Ph); 4.7 (2H, s, C<u>H</u>$_2$Ph); 4.0 (1H, t, C<u>H</u>N).

Example 5

3-(Cyclohexylmethyl)-5-(6-carboxyhexyl)-1-(2-hydroxy-2-cyclohexylethyl amino)hydantoin

By analogy with the combined procedures of Examples 1, 2 and 3, the above compound was prepared.
$^1$H nmr ($CDCl_3$/delta): 4.0 (1H t, C<u>H</u>N); 3.3 (2H m, C<u>H</u>$_2$N).

Example 6

3-(Prop-2-enyl)-5-(6-carboxyhexyl)-1-(2-hydroxy-2-cyclohexylethylamino) hydantoin

By analogy with the combined procedures of Examples 1, 2 and 3, the above compound was prepared.
$^1$H nmr ($CDCl_3$/delta): 6.0-5.0 (3H, m, C<u>H</u>$_2$ = C<u>H</u>); 4.1 (3N, m, C<u>H</u>$_2$N and C<u>H</u>N); 3.3 (1H, m, OC<u>H</u>); 2.9 (2H, m, C<u>H</u>$_2$NH); 2.3 (2H, t, C<u>H</u>$_2$$CO_2$).

Example 7

3-(Prop-2-ynyl)-5-(6-carboxyhexyl)-1-(2-hydroxy-2-cyclohexylethylamino) hydantoin

By analogy with the combined procedures of Examples 1, 2 and 3, the above compound was prepared.
$^1$H nmr ($CDCl_3$/delta): 4.3 (2H, m, C<u>H</u>$_2$N); 4.0 (1H, t, C<u>H</u>N).

Biological Activity

Antagonism of the effects of $PGD_2$ and 5-(6-carboxyhexyl)-1-(3-cyclohexyl -3-hydroxypropyl)hydantoin, a known $PGD_2$ analogue hereinafter referred to as "compound A", by the compounds of Examples 3 to 5 was studied in human washed platelets and rabbit jugular vein.

1. Washed human platelets

Washed platelet suspensions were prepared according to a modified version of the method of Vargas *et al* (Prostaglandins (1982) 23, 929). The suspension was then stored at 4°C for 2 hours and, before use, the following additions were made: $Ca^{++}$, 1μM, indomethacin, 5 mcg $ml^{-1}$; and fibrinogen, 400 mcg $ml^{-1}$. Aggregation responses were measured using 0.5ml aliquots of the suspension maintained at 37°C and

stirred with a metal bar at 900 r.p.m. in 300 BD-S Payton dual channel aggregometers and recorded on Gould BS 272 pen recorders.

Inhibition of platelet aggregation by $PCD_2$ and compound A were studied as follows. The prostaglandin receptor agonists were incubated in the platelet suspension for 6 minutes prior to adding ADP ($5 \times 10^{-5}$ M). The reproducibility of the ADP-induced aggregation response was checked at intervals throughout the experiment. Antagonists were added 2 minutes before the agonist. Responses were expressed as percent inhibitions of the standard ADP-induced aggregation.

2. Rabbit jugular vein

Vascular smooth muscle relaxation in response to $PGD_2$ and compound A was measured as changes in isometric force. Isolated ring segments of rabbit jugular vein suspended in Krebs' buffer, gassed with 95% $O_2$/5% $CO_2$ and maintained at 37°C, were treated with: indomethacin, 1 μg $ml^{-1}$, and the stable thromboxane receptor antagonist BM13177 (Patscheke et al, Biomed. Biochem. Acta (1984) 43, 5321), 30μM. The experiments followed a paired curve design.

Initially, the jugular vein ring segments were subjected to a force of 0.75g, then allowed to stabilize for a period of 60 minutes during which time the preparations were washed three times. Subsequently histamine ($10^{-6}$ M) was added to produce tone. After establishing a stable contraction, relaxation/concentration effect curves were obtained using prostaglandin receptor agonists. Responses were expressed as per cent relaxations of the histamine-induced force. The preparations were then washed thoroughly and allowed to re-stabilise. The antagonists were then incubated for 60 minutes prior to constriction with histamine ($10^{-6}$ M), followed by a second determination of the prostaglandin agonist concentration effect curve.

Analysis of Data

Measurement of antagonist affinity ($pK_B$) was by Schild analysis.

Results

The results shown in the Table 1 demonstrate that the compounds of Examples 3 to 5 all behave as antagonists at the $PGD_2$ receptor on the human platelet.

In particular, the compound of Example 3, the preferred compound of the invention, behaves as a simple competitive antagonist of $PGD_2$ and compound A in the human washed platelet assay. The shifts of the anti-aggregatory concentration effect curves for $PGD_2$ and compound A accord with a Schild plot slope of unity and a $pK_B$ of 9.26. Inhibition of platelet aggregation by $PGD_2$ and compound A and inhibition of the relaxation effects of $PGD_2$ and compound A in the rabbit jugular vein were antagonised with similar potencies. The compound of Example 3 can therefore be classified as a DP-receptor antagonist. Actions of the compound at prostaglandin receptors other than DP were not observed at concentrations up to 1000x greater than that required for DP receptor affinity.

| Example No | $pK_B$ |
|---|---|
| 3 | 9.26 |
| 4 | 10.0 |
| 5 | 8.5 |

Table 1 - Estimates of equilibrium dissociation constants at the $PGD_2$ receptor in human washed platelets

## Claims

**Claims for the following Contracting States : AT, BE, CH, DE, FR, GB, IT, LI, LU, NL, SE**

**1.** A compound of formula (I)

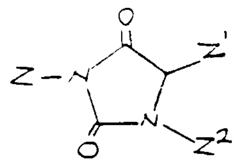

(I)

wherein

Z is $C_{3-12}$ alkenyl or alkynyl, $C_6$ or $C_{10}$ aryl, $C_6$ or $C_{10}$ aryl-$C_{1-12}$ alkyl (wherein in either case the aryl group is optionally substituted by one or more groups independently selected from phenyl, $C_{1-4}$ alkyl, $C_{1-4}$ alkoxy, nitro, and halo) or $C_{4-8}$ cycloalkyl-$C_{1-12}$ alkyl;

$Z^1$ is a group of formula $-CH_2-X-X^1-X^2$, wherein X is selected from $-(CH_2)_2-$ and cis and trans -CH = CH-, $X^1$ is a covalent bond or a straight or branched alkylene chain having from 1 to 6 carbon atoms optionally having one of its methylene groups replaced by oxa (-O-) or thia (-S-) provided that at least one carbon atom separates the oxa or thia group from a carboxyl or -CH = CH- group, and $X^2$ is a carboxyl group, a $C_{1-4}$ alkyl ester, or an amide group;

$Z^2$ is a group selected from $-NH-CH_2-R$ and -N = CH-R, wherein R is a group selected from -CO-Y and $-CH(Y^1)-Y$, Y being a group selected from $C_{3-8}$ alkyl, $C_{3-8}$ alkenyl, phenyl-$C_{1-4}$ alkyl and phenyl (wherein the phenyl group in both cases is optionally substituted by one or more groups independently selected from $C_{1-4}$ alkyl, $C_{1-4}$ alkoxy, nitro, halo and trihalomethyl), $C_{4-8}$ cycloalkyl, pyridyl, thienyl, tetrahydropyranyl and tetrahydrofuryl; and $Y^1$ being a group selected from hydrogen, hydroxy, $C_{1-4}$ alkoxy and $C_{2-5}$ acyloxy;

and salts and solvates thereof.

**2.** A compound of formula (I) as claimed in claim 1, wherein

Z is phenyl or phenyl-$C_{1-3}$ alkyl wherein the phenyl group is optionally substituted by one or more groups independently selected from phenyl, alkyl and alkoxy;

$Z^1$ is $-CH_2-X-X^1-X^2$ wherein X is $-(CH_2)_2-$, $X^1$ is an alkylene chain of 2 to 4 carbon atoms and $X^2$ is a carboxyl group or a $C_{1-4}$ all ester; and

$Z^2$ is a group selected from $-NH-CH_2-R$ and -N = CH-R wherein R is a group of formula $-CH(Y^1)-Y$ in which $Y^1$ is hydroxy and Y is phenyl, $C_{3-8}$ alkyl, or $C_{4-8}$ cycloalkyl;

and salts and solvates thereof.

**3.** A compound of formula (I) as claimed in claim 1, which compound is selected from

3-benzyl-5-(6-carboxyhexyl)-1-(2-cyclohexyl-2-hydroxyethylamino) hydantoin; and

3-benzyl-5-(6-carboxyhexyl)-1-(2-cyclohexyl-2-hydroxyethylidene amino)hydantoin;

and salts and solvates thereof.

**4.** A compound of formula (I) as claimed in any of claims 1 to 3, or a pharmaceutically acceptable salt or solvate thereof, for use in human medical therapy.

**5.** A compound of formula (I) as claimed in any of claims 1 to 3, or a pharmaceutically acceptable salt or solvate thereof, for use in the prophylaxis or treatment of a clinical condition wherein blockade of the DP-receptor mediated effects of $PGD_2$ is indicated.

**6.** A compound of formula (I) as claimed in any of claims 1 to 3, or a pharmaceutically acceptable salt or solvate thereof, for use in the prophylaxis or treatment of a clinical condition associated with an overproduction of $PGD_2$.

**7.** A compound of formula (I) as claimed in any of claims 1 to 3, or a pharmaceutically acceptable salt or solvate thereof, for use in the prophylaxis or treatment of a clinical condition which involves mast cell disfunction.

**8.** A compound of formula (I) as claimed in any of claims 5 to 7, wherein the clinical condition is systemic mastocytis or a systemic mast cell activation disorder, urticaria, or allergic rhinitis.

**9.** A compound of formula (I) as claimed in any of claims 1 to 3, or a pharmaceutically acceptable salt or solvate thereof, for use as a prostanoid receptor probe specific for $PGD_2$ receptors.

**10.** Use of a compound of formula (I) as claimed in any of claims 1 to 3, or a pharmaceutically acceptable salt or solvate thereof, in the manufacture of a therapeutic agent for the prophylaxis or treatment of a clinical condition wherein blockade of the DP-receptor mediated effects of $PGD_2$ is indicated.

**11.** Use of a compound of formula (I) as claimed in any of claims 1 to 3, or a pharmaceutically acceptable salt or solvate thereof, in the manufacture of a therapeutic agent for the prophylaxis or treatment of a clinical condition associated with an overproduction of $PGD_2$.

**12.** Use of a compound of formula (I) as claimed in any of claims 1 to 3, or a pharmaceutically acceptable salt or solvate thereof, in the manufacture of a therapeutic agent for the prophylaxis or treatment of a clinical condition which involves mast cell disfunction.

**13.** Use of a compound of formula (I) as claimed in any of claims 10 to 12, wherein the clinical condition is systemic mastocytosis or a systemic mast cell activation order, urticaria, or allergic rhinitis.

**14.** Use of a compound of formula (I) as claimed in any of claims 1 to 3, or a pharmaceutically acceptable salt or solvate thereof, in the manufacture of a reagent which is suitable as a prostanoid receptor probe specific for $PGD_2$ receptors.

**15.** A pharmaceutical formulation comprising a compound of formula (I) as claimed in any of claims 1 to 3, or a pharmaceutically acceptable salt or solvate thereof, one or more pharmaceutically acceptable carriers and/or excipients therefor, and, optionally, one or more other therapeutic ingredients.

**16.** A process for the preparation of a compound of formula (I) as claimed in any of claims 1 to 3, or a pharmaceutically acceptable salt or solvate thereof, which process comprises

(a) for the preparation of hydantoins wherein $Z^2$ is -N=CH-R, wherein R is as defined above, reacting a compound of formula (II) with a compound of formula (III)

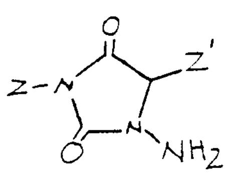

R-HCO (III)

(II)

wherein Z, $Z^1$ and R are as defined above, in the presence of a base in a suitable solvent;

(b) for the preparation of hydantoins wherein $Z^2$ is -N=CH-R, wherein R is as defined above, reacting a compound of formula (IV)

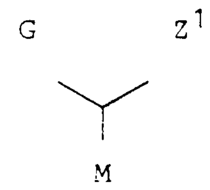

(IV)

wherein $Z^1$ is as defined above, G is carboxy or a derivative thereof or cyano, and M is a leaving group, with a compound of formula (V)

ZHNCONHN=CHR (V)

wherein R and Z are as defined above, in the presence of base in a polar solvent;
(c) for the preparation of hydantoins wherein $Z^2$ is -N=CH-R, wherein R is as defined above, cyclising a compound of formula (VI)

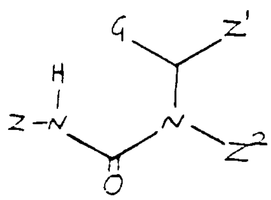

(VI)

wherein G, Z, $Z^1$ and $Z^2$ are as defined above, in the presence of acid or base or by heating in an inert solvent;
(d) for the preparation of hydantoins wherein $Z^2$ is -N=CH-R, wherein R is as defined above, treating a compound of formula (VII)

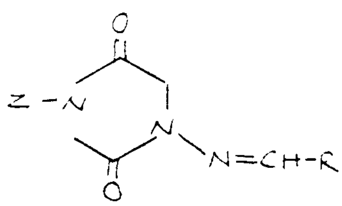

(VII)

wherein R is as defined above, with methyl magnesium carbonate followed by reaction in the presence of base with a compound of formula (VIII)

$M-Z^1$ (VIII)

wherein M and $Z^1$ are as defined above, in a non-protic solvent;

(e) reacting a compound of formula (IX) with a compound of formula (X)

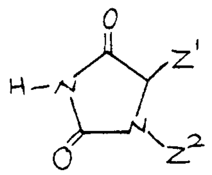

$Z^3$-L (X)

(IX)

wherein $Z^1$ and $Z^2$ are as defined above, L is a suitable leaving group, and $Z^3$ is defined as for Z above (other than $C_6$ or $C_{10}$ aryl) or is an alternative imino protecting group, in the presence of a base and, when L is hydroxy, triphenylphosphate and diethylazodicarboxylate;

and subsequently, if desired, effecting one or more of the following optional conversions in any desired order:

i) where a hydantoin of formula (I) is formed and is an ester, converting said ester into the corresponding acid by hydrolysis under basic conditions;

ii) where a hydantoin of formula (I) is formed and is an acid, converting said acid into the corresponding ester by treatment with the appropriate alcohol;

iii) where a hydantoin of formula (I) is formed wherein Z is hydrogen, converting said hydantoin into a corresponding hydantoin wherein Z is alkyl by treatment with a suitable alkylating agent;

iv) where a hydantoin of formula (I) is formed wherein R is -CH($Y^1$)Y, wherein $Y^1$ is an acyloxy group and Y is as defined above, converting said hydantoin into a corresponding hydantoin of formula (I) wherein $Y^1$ is a hydroxy group by deacylation using a suitable reagent;

v) where a hydantoin of formula (I) is formed wherein R is -CO-Y, converting said hydantoin into a corresponding hydantoin wherein R is -CH(OH)-Y using a suitable reducing agent; or

vi) where a hydantoin of formula (I) is formed wherein $Z^2$ is -N=CH-R, converting said hydantoin into a corresponding hydantoin wherein $Z^2$ is -NH-$CH_2$-R, wherein R is as defined above, using a suitable reducing agent;

and optionally converting the resulting hydantoin into a corresponding salt or solvate by treatment with the appropriate base or solvent.

**Claim for the following Contracting States : ES, GR**

**1.** A process for the preparation of a compound of formula (I)

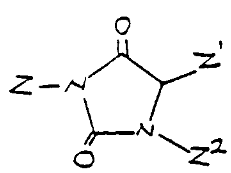

(I)

wherein

Z is $C_{3-12}$ alkenyl or alkynyl, $C_6$ or $C_{10}$ aryl, $C_6$ or $C_{10}$ aryl-$C_{1-12}$ alkyl (wherein in either case the aryl group is optionally substituted by one or more groups independently selected from phenyl, $C_{1-4}$ alkyl, $C_{1-4}$ alkoxy, nitro, and halo) or $C_{4-8}$ cycloalkyl-$C_{1-12}$ alkyl;

$Z^1$ is a group of formula -$CH_2$-X-$X^1$-$X^2$, wherein X is selected from -$(CH_2)_2$- and cis and trans -CH=CH-, $X^1$ is a covalent bond or a straight or branched alkylene chain having from 1 to 6 carbon atoms optionally having one of its methylene groups replaced by oxa (-O-) or thia (-S-) provided that at least one carbon atom separates the oxa or thia group from a carboxyl or -CH=CH- group, and $X^2$ is a carboxyl group, a $C_{1-4}$ alkyl ester, or an amide group;

$Z^2$ is a group selected from -NH-$CH_2$-R and -N=CH-R, wherein R is a group selected from -CO-Y and -CH($Y^1$)-Y, Y being a group selected from $C_{3-8}$ alkyl, $C_{3-8}$ alkenyl, phenyl-$C_{1-4}$ alkyl and phenyl (wherein the phenyl group in both cases is optionally substituted by one or more groups independently selected from $C_{1-4}$ alkyl, $C_{1-4}$ alkoxy, nitro, halo and trihalomethyl), $C_{4-8}$ cycloalkyl, pyridyl, thienyl, tetrahydropyranyl and tetrahydrofuryl; and $Y^1$ being a group selected from hydrogen, hydroxy, $C_{1-4}$ alkoxy and $C_{2-5}$ acyloxy;

and salts and solvates thereof;

which process comprises

(a) for the preparation of hydantoins wherein $Z^2$ is -N=CH-R, wherein R is as defined above, reacting a compound of formula (II) with a compound of formula (III)

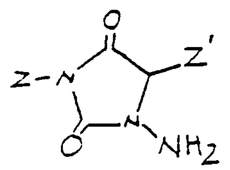

R-HCO (III)

(II)

wherein Z, $Z^1$ and R are as defined above, in the presence of a base in a suitable solvent;

(b) for the preparation of hydantoins wherein $Z^2$ is -N=CH-R, wherein R is as defined above, reacting a compound of formula (IV)

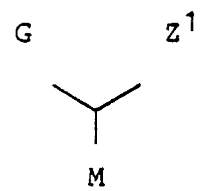

(IV)

wherein $Z^1$ is as defined above, G is carboxy or a derivative thereof or cyano, and M is a leaving group, with a compound of formula (V)

ZHNCONHN=CHR (V)

wherein R and Z are as defined above, in the presence of a base in a polar solvent;

(c) for the preparation of hydantoins wherein $Z^2$ is -N=CH-R, wherein R is as defined above, cyclising a compound of formula (VI)

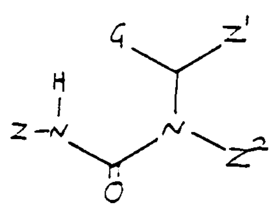

(VI)

wherein G, Z, $Z^1$ and $Z^2$ are as defined above, in the presence of acid or base or by heating in an inert solvent;

(d) for the preparation of hydantoins wherein $Z^2$ is -N = CH-R, wherein R is as defined above, treating a compound of formula (VII)

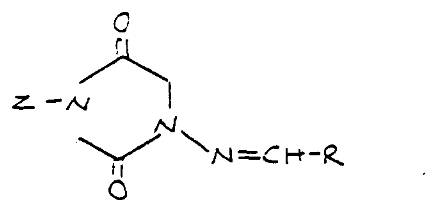

(VII)

wherein R is as defined above, with methyl magnesium carbonate followed by reaction in the presence of base with a compound of formula (VIII)

M-$Z^1$ (VIII)

wherein M and $Z^1$ are as defined above, in a non-protic solvent;

(e) reacting a compound of formula (IX) with a compound of formula (X)

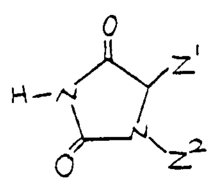

$Z^3$-L (X)

(IX)

wherein $Z^1$ and $Z^2$ are as defined above, L is a suitable leaving group, and $Z^3$ is defined as for Z above (other than $C_6$ or $C_{10}$ aryl) or is an alternative imino protecting group, in the presence of a base and, when L is hydroxy, triphenylphosphate and diethyl azodicarboxylate;

and subsequently, if desired, effecting one or more of the following optional conversions in any desired order:

i) where a hydantoin of formula (I) is formed and is an ester, converting said ester into the corresponding acid by hydrolysis under basic conditions;

ii) where a hydantoin of formula (I) is formed and is an acid, converting said acid into the corresponding ester by treatment with the appropriate alcohol;

iii) where a hydantoin of formula (I) is formed wherein Z is hydrogen, converting said hydantoin into a corresponding hydantoin wherein Z is alkyl by treatment with a suitable alkylating agent;

iv) where a hydantoin of formula (I) is formed wherein R is -CH($Y^1$)Y, wherein $Y^1$ is an acyloxy group and Y is as defined above, converting said hydantoin into a corresponding hydantoin of formula (I) wherein $Y^1$ is a hydroxy group by deacylation using a suitable reagent;

v) where a hydantoin of formula (I) is formed wherein R is -CO-Y, converting said hydantoin into a corresponding hydantoin wherein R is -CH(OH)-Y using a suitable reducing agent; or

vi) where a hydantoin of formula (I) is formed wherein $Z^2$ is -N = CH-R, converting said hydantoin into a corresponding hydantoin wherein $Z^2$ is -NH-$CH_2$-R, wherein R is as defined above, using a suitable reducing agent;

and optionally converting the resulting hydantoin into a corresponding salt or solvate by treatment with the appropriate base or solvent.

**Patentansprüche**
**Patentansprüche für folgende Vertragsstaaten : AT, BE, CH, DE, FR, GB, IT, LI, LU, NL, SE**

1. Verbindung der Formel (I)

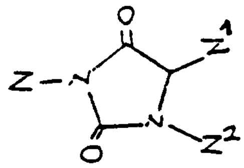

(I)

worin

Z $C_{3-12}$-Alkenyl oder Alkinyl, $C_6$ oder $C_{10}$ Aryl, $C_6$ oder $C_{10}$ Aryl-$C_{1-12}$-Alkyl (wobei in beiden Fällen die Arylgruppe wahlweise substituiert ist durch eine oder mehrere Gruppen, unabhängig ausgewählt aus Phenyl, $C_{1-4}$-Alkyl, $C_{1-4}$-Alkoxy, Nitro und Halo), oder $C_{4-8}$-Cycloalkyl-$C_{1-12}$-Alkyl ist;

$Z^1$ eine Gruppe der Formel $-CH_2-X-X^1-X^2$ ist, worin X ausgewählt ist aus - $(CH_2)_2$- und cis- und trans-CH = CH-, $X^1$ eine kovalente Bindung oder eine geradkettige oder verzweigte Alkylenkette mit 1 bis 6 Kohlenstoffatomen ist, bei der wahlweise eine ihrer Methylengruppen ersetzt ist durch Oxa (-O-) oder Thia (-S-), vorausgesetzt, daß mindestens ein Kohlenstoffatom die Oxa- oder Thiagruppe von einer Carboxyl- oder -CH = CH-Gruppe trennt, und $X^2$ eine Carboxylgruppe, ein $C_{1-4}$-Alkylester oder eine Amidgruppe ist;

$Z^2$ eine Gruppe, ausgewählt aus $-NH-CH_2-R$ und -H = CH-R ist, worin R eine Gruppe, ausgewählt aus -CO-Y und $-CH(Y^1)-Y$ ist, wobei Y eine Gruppe ist, ausgewählt aus $C_{3-8}$-Alkyl, $C_{3-8}$-Alkenyl, Phenyl-$C_{1-4}$-alkyl, und Phenyl (worin die Phenylgruppe in beiden Fällen wahlweise durch eine oder mehrere Gruppen substituiert ist, die unabhängig ausgewählt sind aus $C_{1-4}$-Alkyl, $C_{1-4}$-Alkoxy, Nitro, Halo und Trihalomethyl), $C_{4-8}$-Cycloalkyl, Pyridyl, Thienyl, Tetrahydropyranyl und Tetrahydrofuryl; und $Y^1$ eine Gruppe ist, die ausgewählt ist aus Wasserstoff, Hydroxy, $C_{1-4}$-Alkoxy und $C_{2-5}$-Acyloxy;

und ihre Salze und Solvate.

2. Verbindung der Formel (I), wie in Anspruch 1 beansprucht, worin

Z Phenyl oder Phenyl-$C_{1-3}$-alkyl ist, worin die Phenylgruppe wahlweise durch eine oder mehrere Gruppen substituiert ist, die unabhängig ausgewählt sind aus Phenyl, Alkyl und Alkoxy; und

$Z^1$ $-CH_2-X-X^1-X^2$ ist, worin X $-(CH_2)_2$- ist, $X^1$ eine Alkylenkette von 2 bis 4 Kohlenstoffatomen ist und $X^2$ eine Carboxylgruppe oder ein $C_{1-4}$-Alkylester ist; und

$Z^2$ eine Gruppe ist, ausgewählt aus $-NH-CH_2-R$ und -N = CH-R, worin R eine Gruppe der Formel -CH-$(Y^1)$-Y ist, worin $Y^1$ Hydroxy ist und Y Phenyl, $C_{3-8}$-Alkyl oder $C_{4-8}$-Cycloalkyl ist;

und deren Salze und Solvate.

3. Verbindung der Formel (I), wie in Anspruch 1 beansprucht, welche ausgewählt ist aus

3-Benzyl-5-(6-carboxyhexyl)-1-(2-cyclohexyl-2-hydroxyethylamino)-hydantoin; und

3-Benzyl-5-(6-carboxyhexyl)-1-(2-cyclohexyl-2-hydroxyethylidenamino)-hydantoin;

und deren Salze und Solvate.

4. Verbindung der Formel (I), wie in einem der Ansprüche 1 bis 3 beansprucht, oder eines ihrer pharmazeutisch zulässigen Salze oder Solvate zur Verwendung bei der medizinischen Therapie am

Menschen.

5. Verbindung der Formel (I), wie in einem der Ansprüche 1 bis 3 beansprucht, oder eines ihrer pharmazeutisch zulässigen Salze oder Solvate zur Verwendung bei der Prophylaxe oder Behandlung eines Krankheitszustands, worin eine Blockade der durch den DP-Rezeptor vermittelten Wirkungen von $PGD_2$ indiziert ist.

6. Verbindung der Formel (I), wie in einem der Ansprüche 1 bis 3 beansprucht, oder eines ihrer pharmazeutisch zulässigen Salze oder Solvate zur Verwendung bei der Prophylaxe oder Behandlung eines Krankheitszustands, der mit einer Überproduktion von $PGD_2$ verbunden ist.

7. Verbindung der Formel (I), wie in einem der Ansprüche 1 bis 3 beansprucht, oder eines ihrer pharmazeutisch zulässigen Salze oder Solvate zur Verwendung bei der Prophylaxe oder Behandlung eines Krankheitszustands, an dem Mastzellendysfunktion beteiligt ist.

8. Verbindung der Formel (I), wie in einem der Ansprüche 5 bis 7 beansprucht, worin der Krankheitszustand systemische Mastocytose oder eine systemische Mastzellenaktivierungsstörung, Urtikaria oder allergische Rhinitis ist.

9. Verbindung der Formel (I), wie in einem der Ansprüche 1 bis 3 beansprucht oder eines ihrer pharmazeutisch zulässigen Salze oder Solvate zur Verwendung als für $PGD_2$-Rezeptoren spezifische Prostanoidrezeptorsonde.

10. Verwendung einer Verbindung der Formel (I), wie in einem der Ansprüche 1 bis 3 beansprucht, oder eines pharmazeutisch zulässigen Salzes oder Solvats bei der Herstellung eines therapeutischen Mittels zur Prophylaxe oder Behandlung eines Krankheitszustands, worin die Blockade der durch den DP-Rezeptor vermittelten Wirkungen von $PGD_2$ indiziert ist.

11. Verwendung einer Verbindung der Formel (I), wie in einem der Ansprüche 1 bis 3 beansprucht, oder eines pharmazeutisch zulässigen Salzes oder Solvats davon bei der Herstellung eines therapeutischen Mittels zur Prophylaxe oder Behandlung eines Krankheitszustandes, der mit einer Überproduktion von $PGD_2$ verbunden ist.

12. Verwendung einer Verbindung der Formel (I), wie in einem der Ansprüche 1 bis 3 beansprucht, oder eines pharmazeutisch zulässigen Salze oder Solvates davon bei der Herstellung eines therapeutischen Mittels zur Prophylaxe oder Behandlung eines Krankheitszustands, an dem Mastzellendysfunktion beteiligt ist.

13. Verwendung einer Verbindung der Formel (I), wie in einem der Ansprüche 10 bis 12 beansprucht, worin der Krankheitszustand systemische Mastocytose oder eine systemische Mastzellenaktivierungsstörung, Urtikaria oder allergische Rhinitis ist.

14. Verwendung einer Verbindung der Formel (I), wie in einem der Ansprüche 1 bis 3 beansprucht, oder eines pharmazeutisch zulässigen Salzes oder Solvats davon bei der Herstellung eines Reagens, das als für $PGD_2$-Rezeptoren spezifische Prostanoidrezeptorsonde geeignet ist.

15. Eine pharmazeutische Formulierung, umfassend eine Verbindung der Formel (I), wie in einem der Ansprüche 1 bis 3 beansprucht, oder eines ihrer pharmazeutisch zulässigen Salze oder Solvate, einen oder mehrere pharmazeutisch zulässige Träger und/oder Vehikel dafür und wahlweise einen oder mehrere andere therapeutische Inhaltsstoffe.

16. Verfahren zur Herstellung einer Verbindung der Formel (I), wie in einem der Ansprüche 1 bis 3 beansprucht, oder eines pharmazeutisch zulässigen Salzes oder Solvats davon, welches umfaßt:

(a) zur Herstellung von Hydantoinen, worin $Z^2$ -N=CH-R ist, wobei R wie oben definiert ist, Umsetzen einer Verbindung der Formel (II) mit einer Verbindung der Formel (III)

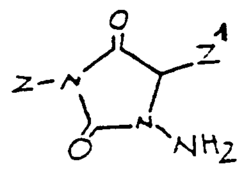

(II)

R-HCO (III)

worin Z, $Z^1$ und R wie oben definiert sind, in Anwesenheit einer Base in einem geeigneten Lösungsmittel;

(b) zur Herstellung von Hydantoinen, worin $Z^2$ -N=CH-R ist, wobei R wie oben definiert ist, Umsetzen einer Verbindung der Formel (IV)

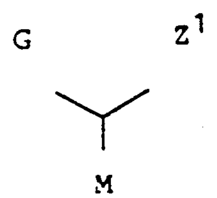

(IV)

worin $Z^1$ wie oben definiert ist, G Carboxy oder ein Carboxyderivat oder Cyano ist und M eine Abgangsgruppe ist, mit einer Verbindung der Formel (V)

ZHNCONHN=CHR (V)

worin R und Z wie oben definiert sind, in Anwesenheit einer Base in einem polaren Lösungsmittel;

(c) zur Herstellung von Hydantoinen, worin $Z^2$ -N=CH-R ist, wobei R wie oben definiert ist, Cyclisieren einer Verbindung der Formel (VI)

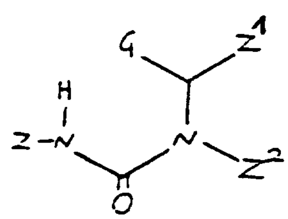

(VI)

worin G, Z, $Z^1$ und $Z^2$ wie oben definiert sind, in Gegenwart einer Säure oder Base oder durch Erhitzen in einem inerten Lösungsmittel;

(d) zur Herstellung von Hydantoinen, worin $Z^2$ -N=CH-R ist, wobei R wie oben definiert ist, das Behandeln einer Verbindung der Formel (VII)

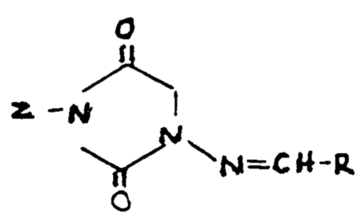

(VII)

worin R wie oben definiert ist, mit Methylmagnesiumcarbonat, gefolgt von Umsetzung in Gegenwart einer Base mit einer Verbindung der Formel (VIII)

$M-Z^1$ (VIII)

worin M und $Z^1$ wie oben definiert sind, in einem nicht-protischen Lösungsmittel;
(e) Umsetzung einer Verbindung der Formel (IX) mit einer Verbindung der Formel (X)

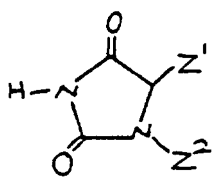

(IX)

$Z^3$-L (X)

worin $Z^1$ und $Z^2$ wie oben definiert sind, L eine geeignete Abgangsgruppe ist und $Z^3$ definiert ist wie Z oben (verschieden von $C_6$ oder $C_{10}$-Aryl) oder eine alternative Iminoschutzgruppe ist, in Anwesenheit einer Base und, wenn L Hydroxy ist, Triphenylphosphat und Diethylazodicarboxylat;

und nachfolgend, falls gewünscht, Durchführung von einer oder mehreren der folgenden wahlweisen Umwandlungen in jeder beliebigen Reihenfolge:
i) wo ein Hydantoin der Formel (I) gebildet wird und ein Ester ist, Umwandeln dieses Esters in die entsprechende Säure durch Hydrolyse unter basischen Bedingungen;
ii) wo ein Hydantoin der Formel (I) gebildet wird und eine Säure ist, Umwandeln dieser Säure in den entsprechenden Ester durch Behandlung mit dem geeigneten Alkohol;
iii) wo ein Hydantoin der Formel (I) gebildet wird, worin Z Wasserstoff ist, Umwandeln dieses Hydantoins in ein entsprechendes Hydantoin, worin Z Alkyl ist, durch Behandlung mit einem geeigneten Alkylierungsmittel;
iv) wo ein Hydantoin der Formel (I) gebildet wird, worin R $-CH(Y^1)Y$ ist, worin $Y^1$ eine Acyloxygruppe ist und Y wie oben definiert ist, Umwandeln dieses Hydantoins in ein entsprechendes Hydantoin der Formel (I), worin $Y^1$ eine Hydroxygruppe ist, durch Deacylierung unter Verwendung eines geeigneten Reagens;
v) wo ein Hydantoin der Formel (I) gebildet wird, worin R -CO-Y ist, Umwandeln dieses Hydantoins in ein entsprechendes Hydantoin, worin R -CH(OH)-Y ist, unter Verwendung eines geeigneten Reduktionsmittels; oder
vi) wo ein Hydantoin der Formel (I) gebildet wird, worin $Z^2$ -N=CH-R ist, Umwandeln dieses Hydantoins in ein entsprechendes Hydantoin, worin $Z^2$ $-NH-CH_2-R$ ist, worin R wie oben definiert ist, unter Verwendung eines geeigneten Reduktionsmittels;
und wahlweise Umwandeln des resultierenden Hydantoins in ein entsprechendes Salz oder Solvat durch Behandlung mit der geeigneten Base oder Lösungsmittel.

**Patentansprüche für folgende Vertragsstaaten : ES, GR**

**1.** Verfahren zur Herstellung einer Verbindung der Formel (I)

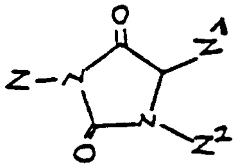

(I)

worin

Z $C_{3-12}$-Alkenyl oder Alkinyl, $C_6$ oder $C_{10}$ Aryl, $C_6$ oder $C_{10}$ Aryl-$C_{1-12}$-Alkyl (wobei in jedem Fall die Arylgruppe wahlweise substituiert ist durch eine oder mehrere Gruppen, welche unabhängig ausgewählt sind aus Phenyl, $C_{1-4}$-Alkyl, $C_{1-4}$-Alkoxy, Nitro und Halo), oder $C_{4-8}$-Cycloalkyl-$C_{1-12}$-Alkyl ist;

$Z^1$ eine Gruppe der Formel $-CH_2-X-X^1-X^2$ ist, worin X ausgewählt ist aus $-(CH_2)_2-$ und cis- und trans- CH = CH-, $X^1$ eine kovalente Bindung oder eine geradkettige oder verzweigte Alkylenkette mit 1 bis 6 Kohlenstoffatomen ist, bei der wahlweise eine ihrer Methylengruppen ersetzt ist durch Oxa (-O-) oder Thia (-S-), vorausgesetzt, daß mindestens ein Kohlenstoffatom die Oxa- oder Thiagruppe von einer Carboxyl- oder -CH = CH-Gruppe trennt, und $X^2$ eine Carboxylgruppe, ein $C_{1-4}$-Alkylester oder eine Amidgruppe ist;

$Z^2$ eine Gruppe, ausgewählt aus $-NH-CH_2-R$ und -N = CH-R ist, worin R eine Gruppe, ausgewählt aus -CO-Y und $-CH(Y^1)-Y$ ist, wobei Y eine Gruppe ist, ausgewählt aus $C_{3-8}$-Alkyl, $C_{3-8}$-Alkenyl, Phenyl-$C_{1-4}$-alkyl und Phenyl (worin die Phenylgruppe in beiden Fällen wahlweise durch eine oder mehrere Gruppen substituiert ist, die unabhängig ausgewählt sind aus $C_{1-4}$-Alkyl, $C_{1-4}$-Alkoxy, Nitro, Halo und Trihalomethyl), $C_{4-8}$-Cycloalkyl, Pyridyl, Thienyl, Tetrahydropyranyl und Tetrahydrofuryl; und $Y^1$ eine Gruppe ist, die ausgewählt ist aus Wasserstoff, Hydroxy, $C_{1-4}$-Alkoxy und $C_{2-5}$-Acyloxy;

und ihrer Salze und Solvate;

wobei das Verfahren umfaßt

(a) zur Herstellung von Hydantoinen, worin $Z^2$ -N = CH-R ist, wobei R wie oben definiert ist, Umsetzen einer Verbindung der Formel (II) mit einer Verbindung der Formel (III)

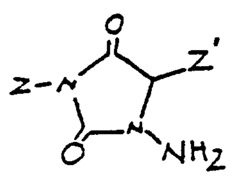

R-HCO (III)

(II)

worin Z, $Z^1$ und R wie oben definiert sind, in Anwesenheit einer Base in einem geeigneten Lösungsmittel;

(b) zur Herstellung von Hydantoinen, worin $Z^2$ -N = CH-R ist, wobei R wie oben definiert ist, Umsetzen einer Verbindung der Formel (IV)

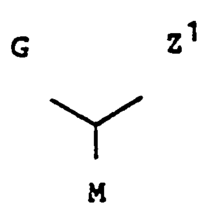

(IV)

worin $Z^1$ wie oben definiert ist, G Carboxy oder ein Carboxyderivat oder Cyano ist und M eine Abgangsgruppe ist, mit einer Verbindung der Formel (V)

ZHNCONHN = CHR (V)

worin R und Z wie oben definiert sind, in Anwesenheit einer Base in einem polaren Lösungsmittel;

(c) zur Herstellung von Hydantoinen, worin $Z^2$ -N = CH-R ist, wobei R wie oben definiert ist, Cyclisieren einer Verbindung der Formel (VI)

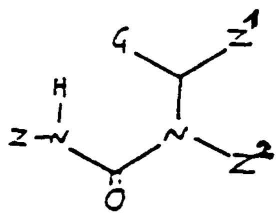

(VI)

worin G, Z, $Z^1$ und $Z^2$ wie oben definiert sind, in Gegenwart einer Säure oder Base oder durch Erhitzen in einem inerten Lösungsmittel;
(d) zur Herstellung von Hydantoinen, worin $Z^2$ -N = CH-R ist, wobei R wie oben definiert ist, das Behandeln einer Verbindung der Formel (VII)

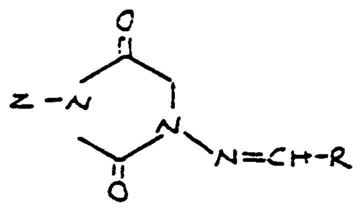

(VII)

worin R wie oben definiert ist, mit Methylmagnesiumcarbonat, gefolgt von einer Umsetzung in Gegenwart einer Base mit einer Verbindung der Formel (VIII)

M-$Z^1$ (VIII)

worin M und $Z^1$ wie oben definiert sind, in einem nichtprotischen Lösungsmittel;
(e) Umsetzung einer Verbindung der Formel (IX) mit einer Verbindung der Formel (X)

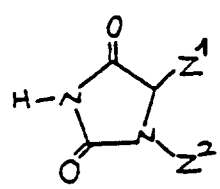

$Z^3$-L (X)

(IX)

worin $Z^1$ und $Z^2$ wie oben definiert sind, L eine geeignete Abgangsgruppe ist und $Z^3$ definiert ist wie Z oben (verschieden von $C_6$- oder $C_{10}$-Aryl) oder eine alternative Iminoschutzgruppe ist, in Anwesenheit einer Base und, wenn L Hydroxy ist, Triphenylphosphat und Diethylazodicarboxylat;

und nachfolgend, falls gewünscht, Durchführung von einer oder mehreren der folgenden wahlweisen Umwandlungen in jeder gewünschten Reihenfolge:

i) wo ein Hydantoin der Formel (I) gebildet wird und ein Ester ist, Umwandeln dieses Esters in die entsprechende Säure durch Hydrolyse unter basischen Bedingungen;
ii) wo ein Hydantoin der Formel (I) gebildet wird und eine Säure ist, Umwandeln dieser Säure in den entsprechenden Ester durch Behandlung mit dem geeigneten Alkohol;
iii) wo ein Hydantoin der Formel (I) gebildet wird, worin Z Wasserstoff ist, Umwandeln dieses Hydantoins in ein entsprechendes Hydantoin, worin Z Alkyl ist, durch Behandlung mit einem

geeigneten Alkylierungsmittel;

iv) wo ein Hydantoin der Formel (I) gebildet wird, worin R $-CH(Y^1)Y$ ist, worin $Y^1$ eine Acyloxygruppe ist und Y wie oben definiert ist, Umwandeln dieses Hydantoins in ein entsprechendes Hydantoin der Formel (I), worin $Y^1$ eine Hydroxygruppe ist, durch Deacylierung unter Verwendung eines geeigneten Reagens;

v) wo ein Hydantoin der Formel (I) gebildet wird, worin R -CO-Y ist, Umwandeln dieses Hydantoins in ein entsprechendes Hydantoin, worin R -CH(OH)-Y ist, unter Verwendung eines geeigneten Reduktionsmittels; oder

vi) wo ein Hydantoin der Formel (I) gebildet wird, worin $Z^2$ -N=CH-R ist, Umwandeln dieses Hydantoins in ein entsprechendes Hydantoin, worin $Z^2$ $-NH-CH_2-R$ ist, worin R wie oben definiert ist, unter Verwendung eines geeigneten Reduktionsmittels;

und wahlweise Umwandeln des resultierenden Hydantoins in ein entsprechendes Salz oder Solvat durch Behandlung mit der geeigneten Base oder Lösungsmittel.

## Revendications

**Revendications pour les Etats contractants suivants : AT, BE, CH, DE, FR, GB, IT, LI, LU, NL, SE**

**1.** Composé de formule (I).

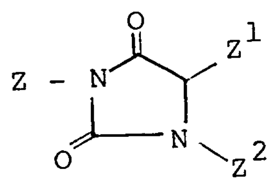

(I)

où

Z est $C_{3-12}$-alcényle ou -alcynyle, $C_6$- ou $C_{10}$-aryle, $C_6$- ou $C_{10}$-aryl-$C_{1-12}$-alcoyle (où dans l'un ou l'autre cas le radical aryle est facultativement substitué par un ou plusieurs radicaux choisis indépendamment parmi phényle, $C_{1-4}$-alcoyle, $C_{1-4}$-alcoxy, nitro et halo) ou $C_{4-8}$-cycloalcoyl-$C_{1-12}$-alcoyle;

$Z^1$ est un radical de formule $-CH_2-X-X^1-X^2$, où X est choisi parmi $-(CH_2)_2-$ et cis et trans -CH=CH-, $X^1$ est une liaison covalente ou une chaîne alcoylène droite ou ramifiée de 1 à 6 atomes de carbone dont l'un des radicaux méthylène est facultativement remplacé par oxa (-O-) ou thia (-S-), avec la condition qu'au moins un atome de carbone sépare le radical oxa ou thia d'un radical carboxyle ou -CH=CH- et $X^2$ est un radical carboxyle, un ester $C_{1-4}$-alcoylique ou un radical amide;

$Z^2$ est un radical choisi parmi $-NH-CH_2-R$ et -N=CH-R, où R est un radical choisi parmi -CO-Y et $-CH(Y^1)Y$, Y étant un radical choisi parmi $C_{3-8}$-alcoyle, $C_{3-8}$-alcényle, phényl-$C_{1-4}$-alcoyle et phényle (où le radical phényle est dans les deux cas facultativement substitué par un ou plusieurs radicaux choisis indépendamment parmi $C_{1-4}$-alcoyle, $C_{1-4}$-alcoxy, nitro, halo et trihalométhyle), $C_{4-8}$-cycloalcoyle, pyridyle, thiényle, tetrahydropyrannyle et tetrahydrofuryle et $Y^1$ étant un radical choisi parmi hydrogène, hydroxyle, $C_{1-4}$-alcoxy et $C_{2-5}$-acyloxy;

et les sels et solvates de celui-ci.

**2.** Composé de formule (I) suivant la revendication 1, où

Z est phényle ou phényl-$C_{1-3}$-alcoyle dont le radical phényle est facultativement substitué par un ou plusieurs radicaux choisis indépendamment par phényle, alcoyle et alcoxy;

$Z^1$ est $-CH_2-X-X^1-X^2$ où X est $-(CH_2)_2-$, $X^1$ est une chaîne alcoylène de 2 à 4 atomes de carbone et $X^2$ est un radical carboxyle ou un ester $C_{1-4}$-acoylique; et

$Z^2$ est un radical choisi parmi $-NH-CH_2-R$ et -N=CH-R, où R est un radical de formule $-CH(Y^1)-Y$ où $Y^1$ est hydroxyle et Y est phényle, $C_{3-8}$-alcoyle ou $C_{4-8}$-cycloalcoyle;

et les sels et solvates de celui-ci.

**3.** Composé de formule (I) suivant la revendication 1, lequel composé est choisi parmi

la 3-benzyl-5-(6-carboxyhexyl)-1-(2-cyclohexyl-2-hydroxyéthylamino)hydantoïne et

la 3-benzyl-5-(6-carboxyhexyl)-1-(2-cyclohexyl-2-hydroxyéthylidèneamino)hydantoïne

et leurs sels et solvates.

**4.** Composé de formule (I) suivant l'une quelconque des revendications 1 à 3, ou un sel ou solvate pharmaceutiquement acceptable de celui-ci, à utiliser en thérapeutique médicale humaine.

**5.** Composé de formule (I) suivant l'une quelconque des revendications 1 à 3, ou un sel ou solvate pharmaceutiquement acceptable de celui-ci, à utiliser dans la prophylaxie ou le traitement d'un état clinique dans lequel le blocage des effets de la $PGD_2$ sous la médiation des récepteurs DP est indiqué.

**6.** Composé de formule (I) suivant l'une quelconque des revendications 1 à 3, ou un sel ou solvate pharmaceutiquement acceptable de celui-ci, à utiliser dans la prophylaxie ou le traitement d'un état clinique associé à une surproduction de la $PGD_2$.

**7.** Composé de formule (I) suivant l'une quelconque des revendications 1 à 3, ou un sel ou solvate pharmaceutiquement acceptable de celui-ci, à utiliser dans la prophylaxie ou le traitement d'un état clinique qui implique le dysfonctionnement des mastocytes.

**8.** Composé de formule (I) suivant l'une quelconque des revendications 5 à 7, dans lequel l'état clinique est la mastocytose systémique ou un désordre d'activation systémique des mastocytes, l'urticaire ou la rhinite allergique.

**9.** Composé de formule (I) suivant l'une quelconque des revendications 1 à 3, ou un sel ou solvate pharmaceutiquement acceptable de celui-ci, à utiliser comme sonde des récepteurs prostanoïdes spécifique pour les récepteurs de la $PGD_2$.

**10.** Utilisation d'un composé de formule (I) suivant l'une quelconque des revendications 1 à 3, ou d'un sel ou solvate pharmaceutiquement acceptable de celui-ci, dans la fabrication d'un agent thérapeutique pour la prophylaxie ou le traitement d'un état clinique dans lequel le blocage des effets de la $PGD_2$ sous la médiation des récepteurs DP est indiqué.

**11.** Utilisation d'un composé de formule (I) suivant l'une quelconque des revendications 1 à 3, ou d'un sel ou solvate pharmaceutiquement acceptable de celui-ci, dans la fabrication d'un agent thérapeutique pour la prophylaxie ou le traitement d'un état clinique associé à une surproduction de $PGD_2$.

**12.** Utilisation d'un composé de formule (I) suivant l'une quelconque des revendications 1 à 3, ou d'un sel ou solvate pharmaceutiquement acceptable de celui-ci, dans la fabrication d'un agent thérapeutique pour la prophylaxie ou le traitement d'un état clinique qui implique le dysfonctionnement des mastocytes.

**13.** Utilisation d'un composé de formule (I) suivant l'une quelconque des revendications 10 à 12, dans laquelle l'état clinique est la mastocytose systémique ou un désordre d'activation systémique des mastocytes, l'urticaire ou la rhinite allergique.

**14.** Utilisation d'un composé de formule (I) suivant l'une quelconque des revendications 1 à 3, ou d'un sel ou solvate pharmaceutiquement acceptable de celui-ci, dans la fabrication d'un réactant qui convient comme sonde des récepteurs prostanoïdes spécifique pour les récepteurs de la $PGD_2$.

**15.** Composition pharmaceutique comprenant un composé de formule (I) suivant l'une quelconque des revendications 1 à 3, ou un sel ou solvate pharmaceutiquement acceptable de celui-ci, un ou plusieurs excipients et/ou diluants pharmaceutiquement acceptables et facultativement un ou plusieurs autres constituants thérapeutiques.

**16.** Procédé de préparation d'un composé de formule (I) suivant l'une quelconque des revendications 1 à 3, ou d'un sel ou solvate pharmaceutiquement acceptable de celui-ci, lequel procédé comprend

(a) pour la préparation d'hydantoïnes où $Z^2$ est $-N=CH-R$, où R est tel que défini ci-dessus, la réaction d'un composé de formule (II) avec un composé de formule (III)

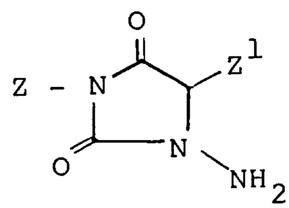

(II)

$R-HCO$

(III)

où Z, $Z^1$ et R sont tels que définis ci-dessus, en présence d'une base dans un solvant approprié;
(b) pour la préparation d'hydantoïnes où $Z^2$ est $-N=CH-R$, où R est tel que défini ci-dessus, la réaction d'un composé de formule (IV)

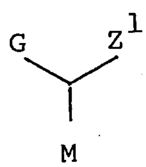

(IV)

où $Z^1$ est tel que défini ci-dessus, G est carboxyle, ou un dérivé de celui-ci, ou cyano, et M est un radical partant, avec un composé de formule (V)

$ZHNCONHN=CHR$ (V)

où R et Z sont tels que définis ci-dessus en présence d'une base dans un solvant polaire;
(c) pour la préparation d'hydantoïnes où $Z^2$ est $-N=CH-R$, où R est tel que défini ci-dessus, la cyclisation d'un composé de formule (VI)

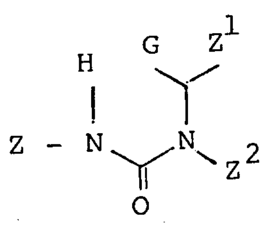

(VI)

où G, Z, $Z^1$ et $Z^2$ sont tels que définis ci-dessus en présence d'un acide ou d'une base ou par chauffage dans solvant inerte;
(d) pour la préparation d'hydantoïnes où $Z^2$ est $-N=CH-R$, où R est tel que défini ci-dessus, la réaction d'un composé de formule (VII)

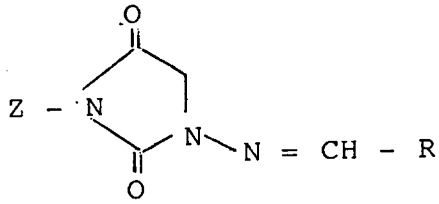

(VII)

où R est tel que défini ci-dessus, avec le carbonate de méthylmagnésium, suivie de la réaction en présence d'une base avec un composé de formule (VIII)

$M-Z^1$ (VIII)

où M et $Z^1$ sont tels que définis ci-dessus, dans un solvant non protique;

(e) la réaction d'un composé de formule (IX) avec un composé de formule (X)

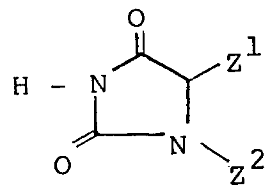

(IX)

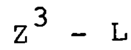

(X)

où $Z^1$ et $Z^2$ sont tels que définis ci-dessus, L est un radical partant approprié, et $Z^3$ et tel que défini à propos de Z ci-dessus (autre que $C_6$- ou $C_{10}$-aryle) ou est un autre radical protecteur de la fonction imino, en présence d'une base et, au cas où L est hydroxyle, de phosphate de triphényle et d'azodicarboxylate de diéthyle;

et ensuite, si la chose est souhaitée, l'exécution d'une ou plusieurs des conversions facultatives suivantes dans tout ordre souhaité

i) lorsqu'une hydantoïne de formule (I) est formée et est un ester, la conversion de cet ester en l'acide correspondant par hydrolyse dans des conditions basiques;

ii) lorsqu'une hydantoïne de formule (I) est formée et est un acide, la conversion de cet acide en l'ester correspondant par traitement avec l'alcool approprié;

iii) lorsqu'une hydantoïne de formule (I) est formée où Z est hydrogène, la conversion de cette hydantoïne en une hydantoïne correspondante où Z est alcoyle par traitement avec un agent d'alcoylation approprié;

iv) lorsqu'une hydantoïne de formule (I) est formée où R est $-CH(Y^1)Y$, où $Y^1$ est un radical acyloxy et Y est tel que défini ci-dessus, la conversion de cette hydantoïne en une hydantoïne correspondante de formule (I) où $Y^1$ est un radical hydroxyle par désacrylation avec un réactant approprié;

v) lorsqu'une hydantoïne de formule (I) est formée où R est -CO-Y, la conversion de cette hydantoïne en une hydantoïne correspondante où R est -CH(OH)-Y avec un agent réducteur approprié; ou

vi) lorsqu'une hydantoïne de formule (I) est formée où $Z^2$ est -N=CH-R, la conversion de cette hydantoïne en une hydantoïne correspondante où $Z^2$ est $-NH-CH_2-R$, où R est tel que défini ci-dessus, avec un agent réducteur approprié;

et facultativement la conversion de l'hydantoïne résultante en un sel ou solvate correspondant par traitement avec la base ou le solvant qui convient.

**Revendication pour les Etats contractants suivants : ES, GR**

**1.** Procédé pour préparer un composé de formule (I)

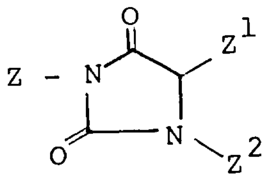

(I)

où

Z est $C_{3-12}$-alcényle ou -alcynyle, $C_6$- ou $C_{10}$-aryle, $C_6$- ou $C_{10}$-aryl-$C_{1-12}$-alcoyle (où dans l'un ou l'autre cas le radical aryle est facultativement substitué par un ou plusieurs radicaux choisis indépendamment parmi phényle, $C_{1-4}$-alcoyle, $C_{1-4}$-alcoxy, nitro et halo) ou $C_{4-8}$-cycloalcoyl-$C_{1-12}$-alcoyle;

$Z^1$ est un radical de formule $-CH_2-X-X^1-X^2$, où X est choisi parmi $-(CH_2)_2-$ et cis et trans -CH=CH-, $X^1$ est une liaison covalente ou une chaîne alcoylène droite ou ramifiée de 1 à 6 atomes de carbone dont l'un des radicaux méthylène est facultativement remplacé par oxa (-O-) ou thia (-S-), avec la condition qu'au moins un atome de carbone sépare le radical oxa ou thia d'un radical carboxyle ou -CH=CH- et

$X^2$ est un radical carboxyle, un ester $C_{1-4}$-alcoylique ou un radical amide;
$Z^2$ est un radical choisi parmi -NH-$CH_2$-R et -N=CH-R, où R est un radical choisi parmi -CO-Y et -CH-($Y^1$)Y, Y étant un radical choisi parmi $C_{3-8}$-alcoyle, $C_{3-8}$-alcényle, phényl-$C_{1-4}$-alcoyle et phényle (où le radical phényle est dans les deux cas facultativement substitué par un ou plusieurs radicaux choisis indépendamment parmi $C_{1-4}$-alcoyle, $C_{1-4}$-alcoxy, nitro, halo et trihalométhyle), $C_{4-8}$-cycloalcoyle, pyridyle, thiényle, tetrahydropyrannyle et tetrahydrofuryle et $Y^1$ étant un radical choisi parmi hydrogène, hydroxyle, $C_{1-4}$-alcoxy et $C_{2-5}$-acyloxy;
et les sels et solvates de celui-ci,

lequel procédé comprend

(a) pour la préparation d'hydantoïnes où $Z^2$ est -N=CH-R, où R est tel que défini ci-dessus, la réaction d'un composé de formule (II) avec un composé de formule (III)

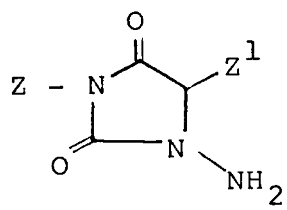

(II)

R - HCO

(III)

où Z, $Z^1$ et R sont tels que définis ci-dessus, en présence d'une base dans un solvant approprié;

(b) pour la préparation d'hydantoïnes où $Z^2$ est -N=CH-R, où R est tel que défini ci-dessus, la réaction d'un composé de formule (IV)

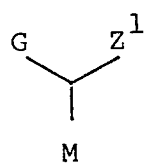

(IV)

où $Z^1$ est tel que défini ci-dessus, G est carboxyle, ou un dérivé de celui-ci, ou cyano, et M est un radical partant, avec un composé de formule (V)

ZHNCONHN=CHR (V)

où R et Z sont tels que définis ci-dessus en présence d'une base dans un solvant polaire;

(c) pour la préparation d'hydantoïnes où $Z^2$ est -N=CH-R, où R est tel que défini ci-dessus, la cyclisation d'un composé de formule (VI)

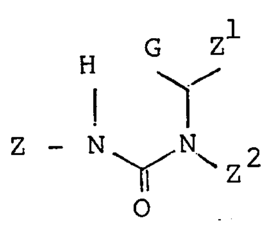

(VI)

où G, Z, $Z^1$ et $Z^2$ sont tels que définis ci-dessus en présence d'un acide ou d'une base ou par chauffage dans solvant inerte;

(d) pour la préparation d'hydantoïnes où $Z^2$ est -N=CH-R, où R est tel que défini ci-dessus, la réaction d'un composé de formule (VII)

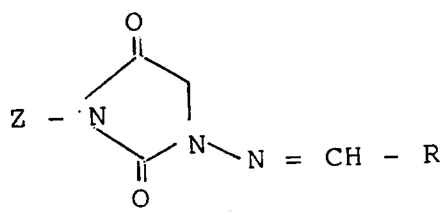

(VII)

où R est tel que défini ci-dessus, avec le carbonate de méthylmagnésium, suivie de la réaction en présence d'une base avec un composé de formule (VIII)

M-$Z^1$ (VIII)

où M et $Z^1$ sont tels que définis ci-dessus, dans un solvant non protique;
(e) la réaction d'un composé de formule (IX) avec un composé de formule (X)

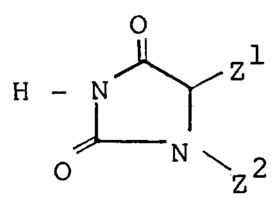

(IX)

$Z^3$ - L

(X)

où $Z^1$ et $Z^2$ sont tels que définis ci-dessus, L est un radical partant approprié, et $Z^3$ et tel que défini à propos de Z ci-dessus (autre que $C_6$- ou $C_{10}$-aryle) ou est un autre radical protecteur de la fonction imino, en présence d'une base et, au cas où L est hydroxyle, de phosphate de triphényle et d'azodicarboxylate de diéthyle;

et ensuite, si la chose est souhaitée, l'exécution d'une ou plusieurs des conversions facultatives suivantes dans tout ordre souhaité

i) lorsqu'une hydantoïne de formule (I) est formée et est un ester, la conversion de cet ester en l'acide correspondant par hydrolyse dans des conditions basiques;

ii) lorsqu'une hydantoïne de formule (I) est formée et est un acide, la conversion de cet acide en l'ester correspondant par traitement avec l'alcool approprié;

iii) lorsqu'une hydantoïne de formule (I) est formée où Z est hydrogène, la conversion de cette hydantoïne en une hydantoïne correspondante où Z est alcoyle par un traitement avec un agent d'alcoylation approprié;

iv) lorsqu'une hydantoïne de formule (I) est formée où R est -CH($Y^1$)Y, où $Y^1$ est un radical acyloxy et Y est tel que défini ci-dessus, la conversion de cette hydantoïne en une hydantoïne correspondante de formule (I) où $Y^1$ est un radical hydroxyle par désacrylation avec un réactant approprié;

v) lorsqu'une hydantoïne de formule (I) est formée où R est -CO-Y, la conversion de cette hydantoïne en une hydantoïne correspondante où R est -CH(OH)-Y avec un agent réducteur approprié; ou

vi) lorsqu'une hydantoïne de formule (I) est formée où $Z^2$ est -N=CH-R, la conversion de cette hydantoïne en une hydantoïne correspondante où $Z^2$ est -NH-$CH_2$-R, où R est tel que défini ci-dessus, avec un agent réducteur approprié;

et facultativement la conversion de l'hydantoïne résultante en un sel ou solvate correspondant par traitement avec la base ou le solvant qui convient.